# EUROPEAN PATENT SPECIFICATION

(11) **EP 4 237 584 B1**
(45) Date of publication and mention of the grant of the patent: **23.04.2025**
(21) Application number: 21805437.7
(22) Date of filing: 29.10.2021
(51) Int. Cl.: C12Q 1/6853, C12Q 1/6858, C12Q 1/6823

(54) **GENERIC CARTRIDGE AND METHOD FOR MULTIPLEX NUCLEIC ACID DETECTION**
GENERISCHE KASSETTE UND VERFAHREN ZUR MULTIPLEX-NUKLEINSÄUREDETEKTION
CARTOUCHE GÉNÉRIQUE ET PROCÉDÉ DE DÉTECTION D'ACIDES NUCLÉIQUES MULTIPLEX

(30) Priority: 29.10.2020 EP 20204806; 17.12.2020 EP 20214901
(43) Date of publication of application: 06.09.2023
(73) Proprietor: Biocartis NV, 2800 Mechelen (BE)
(72) Inventor: DEVOGELAERE, Benoit, 2050 Antwerpen (BE); CLAES, Bart, 9255 Buggenhout (BE); PIOFCZYK, Thomas, 1040 Etterbeek (BE)
(74) Representative: Nederlandsch Octrooibureau
(86) International application number: PCT/EP2021/080221
(87) International publication number: WO 2022/090521

(56) References cited:
- EP-A1- 3 640 340
- EP-A2- 3 453 768
- WO-A1-2009/155271
- WO-A1-2017/013102
- WO-A1-2017/013103
- WO-A1-2020/051521
- WO-A1-2020/165180
- WO-A2-2011/008530
- US-A1- 2009 149 403
- US-A1- 2009 149 403
- US-A1- 2014 315 747
- M. HILVO ET AL: "Novel Theranostic Opportunities Offered by Characterization of Altered Membrane Lipid Metabolism in Breast Cancer Progression", CANCER RESEARCH, vol. 71, no. 9, 1 May 2011 (2011-05-01), pages 3236 - 3245, XP055071277, ISSN: 0008-5472, DOI: 10.1158/0008-5472.CAN-10-3894
- SUPRIYA SWARNKAR: "Kinesin Family of Proteins Kif11 and Kif21B Act as Inhibitory Constraints of Excitatory Synaptic Transmission Through Distinct Mechanisms", SCIENTIFIC REPORTS, vol. 8, no. 1, 27 November 2018 (2018-11-27), US, XP093161325, ISSN: 2045-2322, Retrieved from the Internet <URL:https://www.nature.com/articles/s41598-018-35634-7.pdf> DOI: 10.1038/s41598-018-35634-7
- M. HILVO ET AL: "Novel Theranostic Opportunities Offered by Characterization of Altered Membrane Lipid Metabolism in Breast Cancer Progression", CANCER RESEARCH, vol. 71, no. 9, 1 May 2011 (2011-05-01), pages 3236 - 3245, XP055071277, ISSN: 0008-5472, DOI: 10.1158/0008-5472.CAN-10-3894
- SUPRIYA SWARNKAR: "Kinesin Family of Proteins Kif11 and Kif21B Act as Inhibitory Constraints of Excitatory Synaptic Transmission Through Distinct Mechanisms", SCIENTIFIC REPORTS, vol. 8, no. 1, 27 November 2018 (2018-11-27), US, XP093161325, ISSN: 2045-2322, Retrieved from the Internet <URL:https://www.nature.com/articles/s41598-018-35634-7.pdf> DOI: 10.1038/s41598-018-35634-7
- FALTIN B ET AL: "Mediator probe PCR: a novel approach for detection of real-time PCR based on lavbel-free primary probes and standardized secondary universal fluorogenic reporters", CLINICAL CHEMISTRY, OXFORD UNIVERSITY PRESS, US, vol. 58, 1 November 2012 (2012-11-01), pages 1546 - 1556, XP002694250, ISSN: 0009-9147, [retrieved on 20120824], DOI: 10.1373/CLINCHEM.2012.186734
- MINDIOLA-ROMEROMD ANDRES E. ET AL: "Novel Biocartis Idylla(TM) cartridge-based assay for detection of microsatellite instability in colorectal cancer tissues", EXPERIMENTAL AND MOLECULAR PATHOLOGY., vol. 116, 1 October 2020 (2020-10-01), US, pages 104519, XP055790712, ISSN: 0014-4800, DOI: 10.1016/j.yexmp.2020.104519
- BECHERER LISA ET AL: "Simplified Real-Time Multiplex Detection of Loop-Mediated Isothermal Amplification Using Novel Mediator Displacement Probes with Universal Reporters", ANALYTICAL CHEMISTRY, vol. 90, no. 7, 6 March 2018 (2018-03-06), US, pages 4741 - 4748, XP055791062, ISSN: 0003-2700, Retrieved from the Internet <URL:https://pubs.acs.org/doi/pdf/10.1021/acs.analchem.7b05371> DOI: 10.1021/acs.analchem.7b05371
- KITANO SHIRO ET AL: "A Novel Fully Automated Molecular Diagnostic System (AMDS) for Colorectal Cancer Mutation Detection", PLOS ONE, vol. 8, no. 5, 9 January 2013 (2013-01-09), pages e62989, XP055790683, DOI: 10.1371/journal.pone.0062989
- SIMON WADLE ET AL: "Simplified development of multiplex real-time PCR through master mix augmented by universal fluorogenic reporters", BIOTECHNIQUES, vol. 61, no. 3, 1 January 2016 (2016-01-01), US, pages 123 - 128, XP055527930, ISSN: 0736-6205, DOI: 10.2144/000114443
- JOHNSTON LOUISE ET AL: "Clinical performance evaluation of the Idylla NRAS-BRAF mutation test on retrospectively collected formalin-fixed paraffin-embedded colorectal cancer tissue", vol. 71, no. 4, 12 September 2017 (2017-09-12), GB, pages 336 - 343, XP055790709, ISSN: 0021-9746, Retrieved from the Internet <URL:https://www.ncbi.nlm.nih.gov/pmc/articles/PMC5868529/pdf/jclinpath-2017-204629.pdf> DOI: 10.1136/jclinpath-2017-204629

## Description

### TECHNICAL FIELD

The field of the invention generally relates to detection of nucleic acid targets in a multiplex reaction setting. In particular, disclosed herein are methods for quantifying the number of target nucleic acids in a sample in relation to KIF11 nucleic acids in said sample; methods for determining gDNA contamination in a sample or for determining integrity of nucleic acids in a sample and comprising amplifying KIF11 nucleic acid comprised in the sample using a first and a second KIF11-specific primer pair in a KIF11 amplification reaction, as well as methods for determining gDNA fragmentation or for quality controlling the processing, isolation and amplification process, which comprise generating 3 KIF11 amplicons of discernably different lengths.

### BACKGROUND

There exists a global need for fast and straight-forward development of personalized diagnostic assays that allow to monitor genetically-versatile disease states like infections, organ transplant rejection or cancer in individuals. Such assays should be based on patient-specific genetic information, provide a high reliability and remain cost-effective. The currently existing methods are too time- and resource-intensive, and consequently fail to provide a suitable solution.

In the cancer field, already since the first years of the 21^{st} century, we are observing a paradigm shift from cancer-type specific diagnosis and treatment to a more individualized approach to diagnosis and pan-cancer treatment with a strong focus on individual's underlying genetics and immune response (Hanahan and Weinberg, 2011, Cell 144:646-674). It is consequently now being more broadly recognized that cancer is not one uniform disease but an umbrella term for many different genetically determined acquired pathological proliferation syndromes, wherein not only every cancer type but even every individual cancer appears to have a unique genetic mutational profile (Ciriello et al., 2013, Nat Gen 45:1127-1133). Consequently, despite the still very frequent prevalence of detectable and therapeutically-targetable driver mutations such as those in the *KRAS, BRAF,* or *EGFR* genes in many cancers, there exists a substantial number of remaining cancer cases, where individually-focused testing could greatly benefit patients' management and outcomes.

One way of performing such individualized screening is by Next Generation Sequencing (NGS). Despite NGS analyses are gradually becoming more affordable, they are still relatively expensive and require expert involvement for data interpretation, which is usually out of reach for general practitioners. Also, waiting times for obtaining the NGS results are still substantial. Consequently, NGS is, and for a while will likely still remain, a largely unsuitable technique for regular or periodical follow-up cancer care. Given the speed, sensitivity, ease-of-use, and availability of the existing fully automated systems, the possibly best solution could be provided by qPCR-based detection of selected targets. Furthermore, these fully automated systems enable global deployment of the tests, which brings the test closer to the patient and ensures global patient access. Although excellent mutation-panel-specific diagnostic tests are readily available for cancer patients from several providers, including Biocartis NV, with the state of the art assay development pipelines, provision of personalized custom target-specific cartridges is below profitability margin for the manufacturers. Despite the limited funding and return on investment considerations, bringing a new diagnostic cartridge under present development procedures is still associated with the standard development and manufacturing lead times that are too long from an individual's perspective. Consequently, there exists an urgent need to transform cancer follow-up care for individuals by developing personalized-genetic assays much faster and cheaper, especially for molecular surveillance testing, post-surgical and minimal residual disease (MRD) monitoring.

With the sudden hit of the global Covid-pandemic in 2020, causing lockdowns imposed on many manufacturing companies, which further contributed to extended time-lines and time-to-market periods, and substantially limiting access of many patients to healthcare and disease monitoring systems, it became apparent that a new strategy is needed more than ever for faster and more cost-effective development of custom qPCR-detection-based tests. In particular, in view of the emergence of SARS-CoV-2 as a completely new viral and constantly mutating pathogen, making it challenging to detect, it was particularly desirable for such new strategies to be generally applicable to detection of sequences from other than human organisms and to be extremely customizable and readily adaptable to include detection of rapidly changing sequences.

Hilvo et al., 2011 ("Novel Theranostic Opportunities Offered by Characterization of Altered Membrane Lipid Metabolism in Breast Cancer Progression", CANCER RESEARCH, vol. 71, no. 9, 1 May 2011, pages 3236-3245, XP055071277, ISSN: 0008-5472, DOI: 10.1158/0008-5472.CAN-10-3894) discloses siRNA inhibition of KIF11 as the reference for cell viability assays. Swarnkar et al., 2018 ("Kinesin Family of Proteins Kif11 and Kif21 B Act as Inhibitory Constraints of Excitatory Synaptic Transmission Through Distinct Mechanisms", SCIENTIFIC REPORTS, vol. 8, no. 1,27 November 2018, XP093161325, US ISSN: 2045-2322, DOI: 10.1038/s41598-018-35634-7) discloses determination of KIF11 expression levels using a real time assay.

### SUMMARY

The invention is set out in the appended set of claims.

In some embodiments, the systems or methods disclosed herein comprise a reference system, including a reference target nucleic acid sequence, comprising at least a portion of the KIF11 gene sequence, including the non-coding region of the KIF11 gene. In some embodiments, the reference system comprises one or more of SEQ ID NO:s 69-80, preferably a primer pair selected from the following: SEQ ID NO:s 69 and 70; SEQ ID NO:s 71 and 72; SEQ ID NO:s 73 and 74; SEQ ID NO:s 75 and 76; SEQ ID NO:s 77 and 78; and SEQ ID NO:s 79 and 80; preferably the amplified region is detected by probes selected from SEQ ID NO:s 81-86, preferably via generic reporter selected from SEQ ID NO: 87 and 88.

In some embodiments, the disclosed systems, methods, kits, and components thereof provide superior results compared to prior art methods. By way of example, but not by way of limitation, in some embodiments, the disclosed systems, methods, kits, and components are cheaper (such as vs NGS), easier to use (such as vs any other approach, e.g. qPCR on a plate based system or NGS), and/or faster to design and market (such as vs any other approach, see above).

In a yet another general aspect, uses of the methods, kits, components thereof, and their respective embodiments as described in the continuation, are provided for advantageous applications, which for example comprise but are not limited to detecting multiple genetic targets, possibly in a sample obtained from a patient. The patient can be a cancer patient, a patient suffering from an infectious disease, a transplant receiver, or an expecting future mother. If the patient is a cancer patient, advantageous uses of the disclosed methods, kits, and components include but are not limited to post-NGS analysis patient surveillance, response to treatment or therapy monitoring, minimal residual disease (MRD) detection or monitoring, post-surgery follow up, or in individualized cancer neoantigen-targeting immunotherapy selection.

In an aspect, a method is disclosed for quantifying the number of target nucleic acids in a sample in relation to KIF11 nucleic acids in said sample, comprising:
1. amplifying KIF11 nucleic acids comprised in the sample using a KIF11-specific primer pair in a KIF11 amplification reaction,
   i. wherein each of the members of the KIF11-specific primer pair is -independently from each other- complementary to a KIF11 region located in an exon, intron, or the non-coding sequence of the KIF11 gene;
   ii. wherein the KIF11 amplification reaction is performed in the presence of a KIF11 detectable probe;
2. detecting a signal produced by the KIF11 detectable probe in the KIF11 amplification reaction;
3. quantifying the signal of 2.;
4. amplifying the target nucleic acids from the sample using a target-specific primer pair in a target amplification reaction;
   i. wherein the target amplification reaction is performed in the presence of a target detectable probe; and
5. detecting a signal produced by the target detectable probe in the target amplification reaction;
6. quantifying the signal of 5.;
7. normalizing the quantified signal of 6. to the quantified signal of 3., thereby quantifying the number of target nucleic acids in a sample in relation to KIF11 nucleic acids in the sample.

In yet a further aspect a method is disclosed for determining gDNA contamination in a sample, comprising:
1. amplifying KIF11 nucleic acid comprised in the sample using a first and a second KIF11-specific primer pair in a KIF11 amplification reaction,
   a. wherein at least one member of the first KIF11-specific primer pair is complementary to a KIF11 intron or the non-coding sequence of the KIF11 gene;
   b. wherein the amplification reaction using the first KIF11 specific primer pair is performed in the presence of a first KIF11 detectable probe;
   c. wherein each of the members of the second KIF11-specific primer pair is located in a KIF11 exon;
   d. wherein the amplification reaction using the second KIF11-specific primer pair is performed in the presence of a second KIF11 detectable probe;
2. detecting signals produced by the first and second KIF11 detectable probes in the first and second KIF11 amplification reactions;
3. quantifying the signals of the first and second KIF11 amplification reactions;
4. normalizing the quantified signal of the first amplification reaction to the quantified signal of the second amplification signal, thereby determining gDNA contamination in the sample, preferably the sample is a mitochondrial DNA, cDNA, mRNA, rRNA, tRNA, hnRNA, microRNA, IncRNA, cfDNA, cell-free tumor DNA or an siRNA sample.

In yet a further aspect a method is disclosed for determining integrity of nucleic acids in a sample, comprising:
1. amplifying KIF11 nucleic acid comprised in the sample using a first and a second KIF11-specific primer pair in a KIF11 amplification reaction,
   a. wherein each of the members of the first KIF11-specific primer pair is -independently from each other- complementary to a KIF11 region located in an exon, intron, or the non-coding sequence of the KIF11 gene;
   b. wherein the amplification reaction using the first KIF11 specific primer pair is performed in the presence of a first KIF11 detectable probe;
   c. wherein each of the members of the second KIF11-specific primer pair is - independently from each other- complementary to a KIF11 region located in an exon, intron, or the non-coding sequence of the KIF11 gene;
   d. wherein the amplification reaction using the second KIF11-specific primer pair is performed in the presence of a second KIF11 detectable probe;
   e. preferably, the amplicons generated by the amplification reaction of the first and second KIF11-specific primer pairs are sufficiently far located to not interfere, e.g. the amplicons are at least 600 basepairs (bp), such as at least 700 bp, 800 bp, 900 bp or even more, such as at least 1 kilobasepairs;
2. determining the threshold values in each of said nucleic acid amplification reactions by
   ∘ measuring, at a plurality of different times during the amplification reaction, at least one signal whose intensity is related to the quantity of a nucleic acid sequence being amplified in the reaction;
   ∘ determining the cycle number associated with the characteristic of the derivative, which represents the threshold value;
3. comparing the threshold values (threshold cycle numbers) of the first and second KIF11 amplification reactions;
4. wherein the difference between the threshold cycle numbers (ΔCq) of the first and the second KIF11 amplification reactions is a measure for the integrity of genomic DNA;
   optionally step 2 can comprise
   ∘ deriving a growth curve from the measurements of the signal;
   ∘ calculating a derivative of the growth curve and identifying a characteristic of the derivative;
   ∘ calculating a second derivative of the growth curve, and wherein the characteristic comprises a positive peak of the second derivative; and

In yet a further aspect a method is disclosed for determining gDNA fragmentation, comprising
- generating 3 (first, second and third) KIF11 amplicons of discernably length by first, second and third amplification reactions, preferably by PCR;
- determining the Cq values of said first, second and third amplification reactions;
- comparing the Cq of said first, second and third amplification reactions;
- wherein the differences in Cq values between said first, second and third amplification reactions is an indication of the gDNA fragmentation.

In yet a further aspect a method is disclosed for determining gDNA fragmentation, but which can also be used for assessing contamination of cell-free DNA or cell-free tumor DNA with more intact genomic DNA derived from white blood cells, comprising
- generating a first KIF11 amplicon ("short") by a first amplification reaction; and
- generating a second KIF11 amplicon ("long") by a second amplification reaction;
- determining the Cq values of said first and said second amplification reaction;
- determining the ΔCq value of said first and said second amplification reaction;
- wherein the ΔCq is an indication of gDNA fragmentation.

In yet a further aspect use to amplify a region of the genomic reference gene KIF11 of a kit is disclosed comprising primers and instructions comprising an amplification protocol and analysis of the results, wherein the primers are a primer pair selected from the following: SEQ ID NO:s 69-80, preferably a primer pair selected from the following: SEQ ID NO:s 69 and 70; SEQ ID NO:s 71 and 72; SEQ ID NO:s 73 and 74; SEQ ID NO:s 75 and 76; SEQ ID NO:s 77 and 78; and SEQ ID NO:s 79 and 80; preferably the amplified region is detected by probes selected from SEQ ID NO:s 81-86, preferably via generic reporter selected from SEQ ID NO: 87 and 88.

In yet a further aspect a method is disclosed of quality controlling the processing, isolation and amplification process comprising:
- sample processing;
- nucleic acid isolation;
- generating 3 KIF11 amplicons of discernably different lengths by amplification reactions;
- determining the Cq values of each of said KIF11 amplicons;
wherein the Cq values of the KIF11 amplicons are a measure for the quality control of the processing, isolation and amplification.

In yet a further aspect a kit is disclosed, said kit comprising:
(a) at least one probe designed to anneal to an amplicon of the KIF11 gene;
(b) products and reagents required to carry out the annealing reaction; and
(c) instructions for use;
wherein the probe is a probe, of which at least a part is specific to the amplicons as described herein.

In yet a further aspect a system is disclosed for the automated processing of a biological sample, said system comprising:
- an enclosure configured to contain one or more sample processing modules, each sample processing module configured to hold a removable cartridge as described herein, where said system is configured to operate the sample processing modules to perform PCR to determine the presence and/or quantity of one or more target genes and optionally to determine the level of one or more target DNA sequences within a corresponding removable sample cartridge, wherein said processing on a sample within the corresponding removable sample cartridge performs a method comprising:
- providing a sample in an entry port for receiving biological sample of said cartridge; and using said cartridge:
- means for isolating nucleic acid from the biological sample received in the entry port, said means capable of entering in fluid communication with said entry port for receiving the biological sample;
- a plurality of chambers containing reagents and/or buffers for performing PCR in fluid connection with and positioned downstream of the said entry port for receiving biological sample;
- said plurality of chambers comprising a chamber containing a PCR mix;
- said plurality of chambers comprising at least one chamber containing primers for amplifying all or a region of KIF11 gene; and
- said plurality of chambers comprising a chamber containing a probe for detecting all or a region of said KIF11 gene.
- performing a nucleic acid amplification in said chamber to detect and/or quantify said target nucleic acid, wherein said KIF11 gene is used as an internal control, thereby detecting and/or quantifying said target nucleic acid.

### DESCRIPTION OF FIGURES

For a fuller understanding, reference is made to the following detailed description taken in conjunction with the accompanying figures in which:

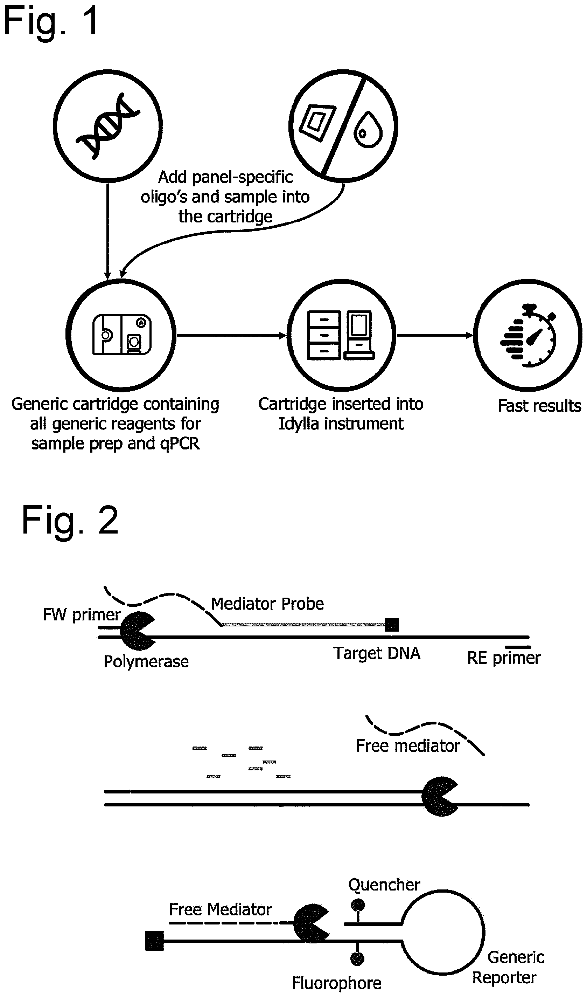

**Figure 1****:** shows the general workflow in which all generic reagents (*i.e.* reagents that are not panel-specific) are present inside a generic cartridge, while the panel-specific reagents and the sample are added by a user through a sample entry port of the generic cartridge;
**Figure 2****:** shows the reaction mechanism of the mediator probe PCR. Extension of the forward (FW) primer by a polymerase leads to hydrolysis of the target specific component of the mediator probe and liberation of the free mediator. In a next step, the free mediator can bind to a generic reporter. Note that the fluorophore and quencher of the generic reporter can be swapped (not shown). Upon extension of the free mediator, a fluorescent signal is created by displacement of the quencher or fluorophore modification and/or hydrolysis of the quencher or fluorophore-linked nucleotides (not shown). Note that the non-hydrolysed mediator probe and generic reporter cannot be extended by the polymerase (as symbolically indicated by a square); RE primer is reverse primer.

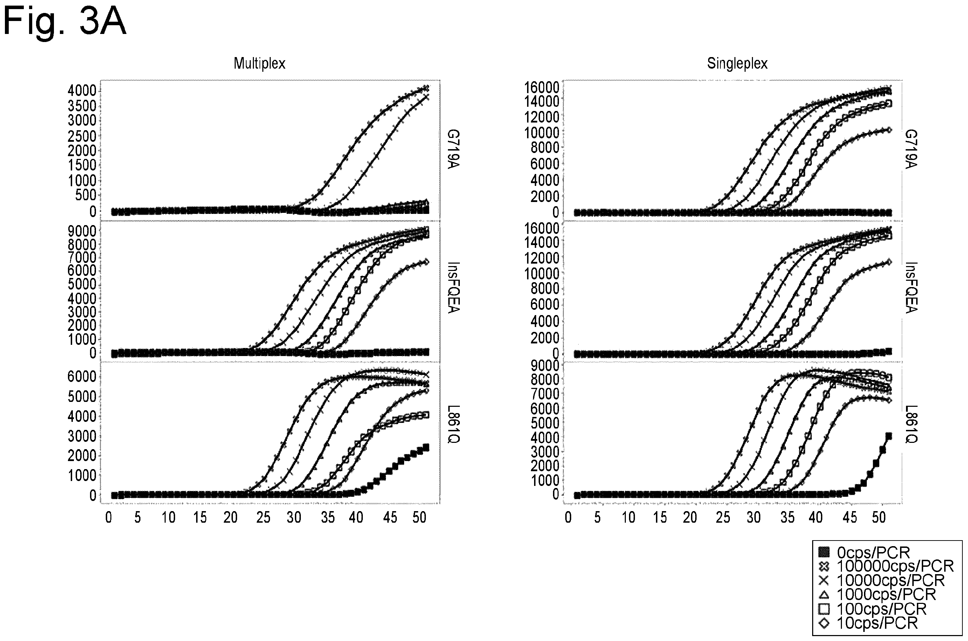

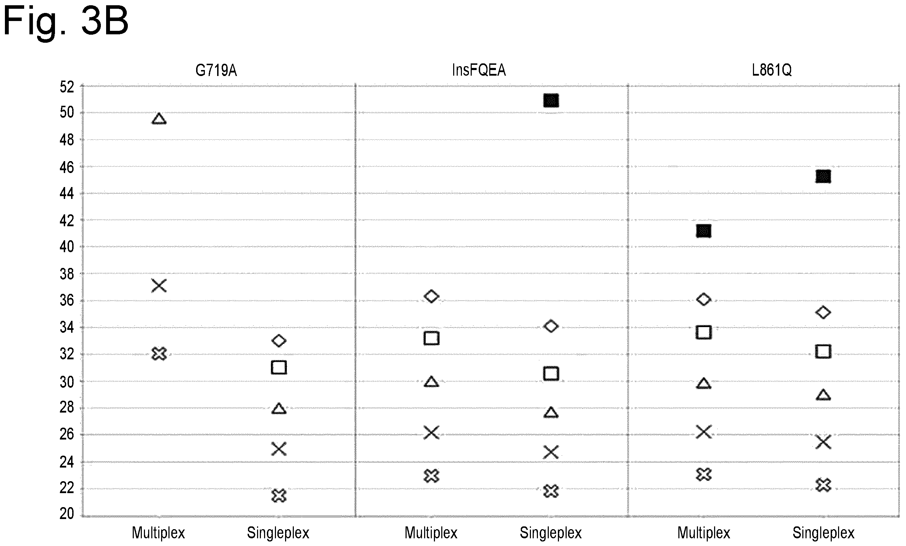

**Figure 3****:** shows the performance for mutation detection using ARMS primers in a singleplex (*i.e.* containing only the primers that are needed to amplify 1 target) as well as in a multiplex (*i.e.* containing primers that are needed to amplify multiple targets) in a 96-well format qPCR instrument. The targets are added as synthetic mutant targets (EGFR G719A; EGFR InsFQEA, EGFR L861Q) at various concentrations in the PCR reaction (which always contains 10,000 copies of genomic wild-type DNA, as well as the oligonucleotide mixture of primers and mediator probes and generic reporters, and the polymerase, dNTPs and PCR salts) as indicated in the legend. Panel A shows the raw curves, panel B shows the Cq values; X-axis: number of PCR cycles, Y-axis: arbitrary fluorescence units;. Solid squares represent the condition where there are 10,000 copies of genomic wild-type DNA present, but no synthetic mutant targets are added.

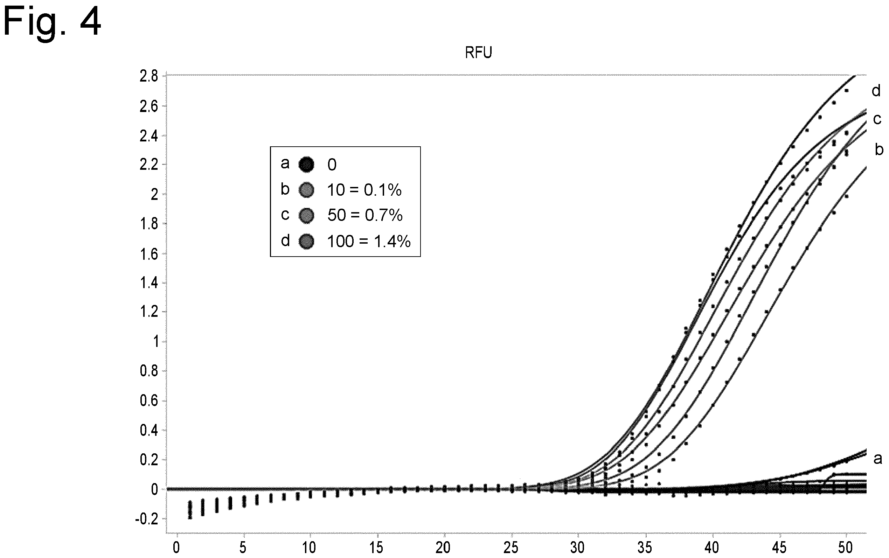

**Figure 4****:** shows the performance for mutation detection using ARMS primers in a multiplex (*i.e*. with an oligonucleotide mixture containing primers that are needed to amplify multiple targets, as well as mediator probes) in an integrated sample-to-result instrument. The targets are added as synthetic mutant targets at various concentrations through the sample entry port, together with the oligonucleotide mixture containing primers and mediator probes, and an formalin-fixed paraffin-embedded (FFPE) clinical sample that was upfront quantified to contain about 7,000 copies of genomic DNA per PCR chamber. The generic reporters, polymerase and dNTPs are spotted in the different chambers of the cartridge. Mutant (synthetic target): 100 (1.4%) - 50 (0.7%) - 10 (0.1%) - 0 (0.0%) copies per PCR. The PCR salts are part of a liquefaction buffer that is generic and present in one of the reagent containers of the cartridge. RFU = Relative Fluorescence Units, *i.e.* the signal obtained from the qPCR component of the integrated sample-to-result instrument;

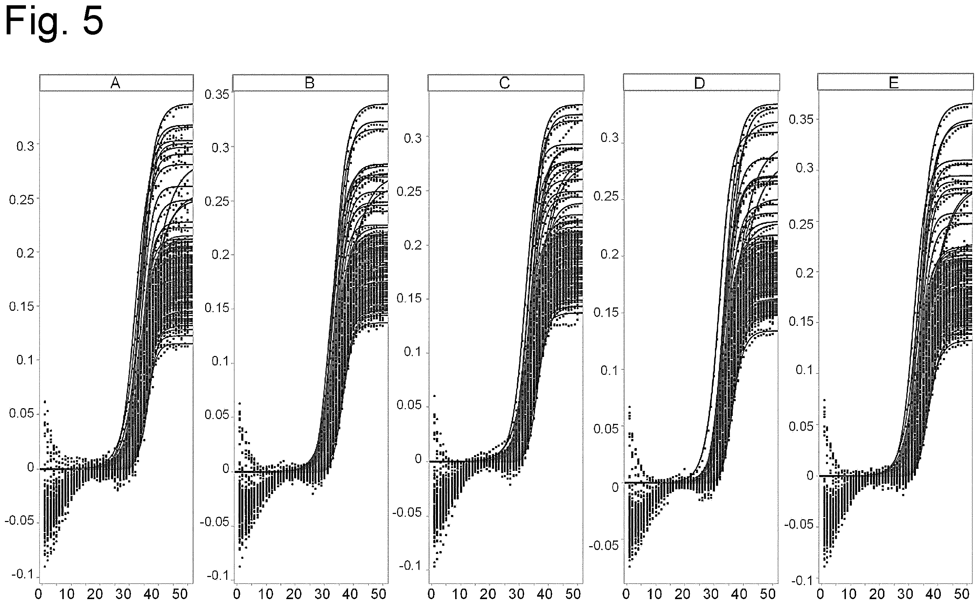

**Figure 5****:** shows the performance for detection of wild-type genomic DNA in a multiplex (*i.e.* with an oligonucleotide mixture containing primers that are needed to amplify multiple targets, as well as mediator probes) in an integrated sample-to-result instrument. The oligonucleotide mixture containing primers and mediator probes, and either an FFPE clinical sample or extracted DNA were added through the sample entry port. The generic reporters, polymerase and dNTPs were spotted in the different chambers of the cartridge. The PCR salts were part of a liquefaction buffer that is generic and present in one of the reagent containers of the cartridge. Y-axis represents signal intensity, X-axis represents cycle number, A-E represents a different PCR compartment;

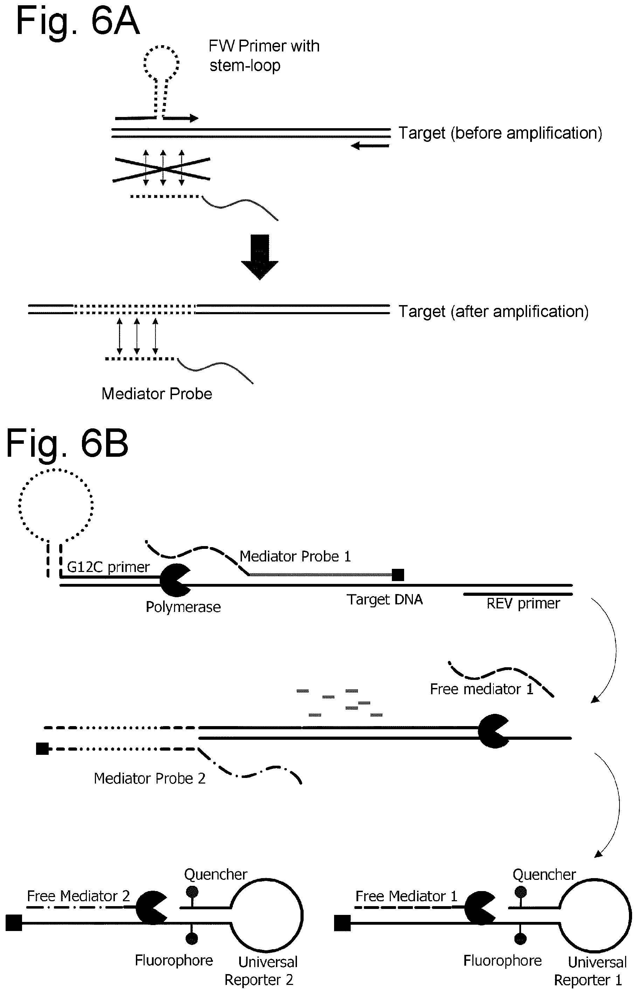

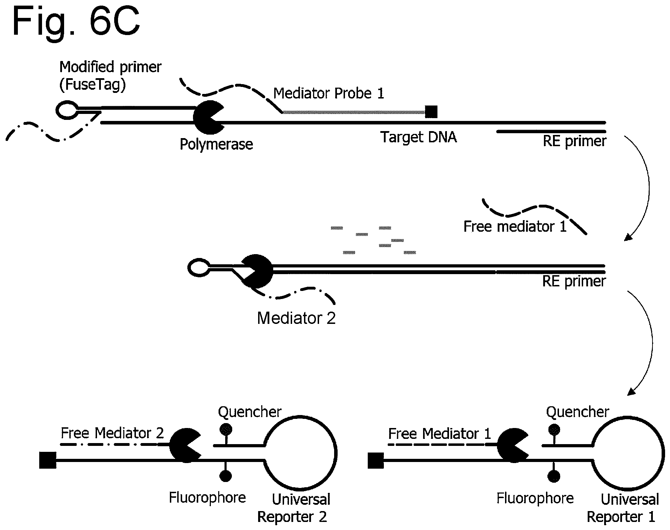

**Figure 6****:** shows concepts enabling the discrimination of neighboring markers. The ARMS primer now includes a stem-loop structure, in which the stem can optionally be composed of the target sequence. Panel A shows one design of an ARMS primer with a stem-loop structure wherein the 3'-end of the primer comprises a sequence specific to the target sequence. The binding site of the mediator probe overlaps (at least partially) with the ARMS primer (indicated as "FW primer with stem-loop"). When the target is not amplified, the mediator probe cannot bind to the target sequence and hence cannot create a signal. The mediator probe also cannot bind to the ARMS primer, as it is in a stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal; Panel B shows an alternative design of an ARMS primer with a stem-loop structure wherein the stem loop-structure defines the 5' end of the primer and can be used as a generic tail tag. Similar to Panel A, the binding site of mediator probe 2 overlaps (at least partially) with the ARMS primer (indicated as "G12C primer"). When the target is not amplified, the mediator probe 2 cannot bind to the target sequence and hence cannot create a signal. Mediator probe 2 also cannot bind to the ARMS primer (G12C primer), as it is in a stem-loop configuration and hence inaccessible for the mediator probe. When the target is amplified, the stem structure can be unfolded by the polymerase, thereby creating a binding site for mediator probe. Once bound, the free mediator is released as the other primer is extended, and the free mediator can bind to the spotted generic reporter and create a signal. Presence of mediator probe 1 is facultative, but can be used as a positive control and/or to increase options to detect the target. The mediator probes consist 5' to 3' of a first portion, wherein the first portion comprises a unique generic sequence tag ("UGST") complementary to the UGST binding site of the corresponding generic reporter molecule (universal reporter), a second portion, wherein the second portion is complementary to the target (mediator probe 1) or complementary to the ARMS primer (mediator probe 2) and a polymerase extension blocker (indicated by the square box). The generic reporter molecules (universal reporter) are singled-stranded DNA, and comprise: i)a first member of a fluorophore/quencher pair; ii) a stem-loop structure; iii) a second member of a fluorophore/quencher pair; iv) a unique generic sequence tag ("UGST") binding site; and v) a polymerase extension blocker. Panel C shows the "FuseTag" concept enabling the discrimination of neighboring markers. Compared to Panel B, the forward ARMS primer is modified ("FuseTag" primer) now including from 5' to 3': a first portion, wherein the first portion comprises a unique generic sequence tag ("UGST"), which when released is indicated as free mediator 2 in Figure 6C; a stem-loop structure; and a second portion, wherein the second portion is complementary to the target (and preferably allele-specific). When the specific target is not amplified by the FuseTag primer, the unique generic sequence tag (mediator 2) is not released and hence cannot create a signal. However, the mediator probe 1 can still be hydrolyzed by another forward primer present in the multiplex reaction mixture.

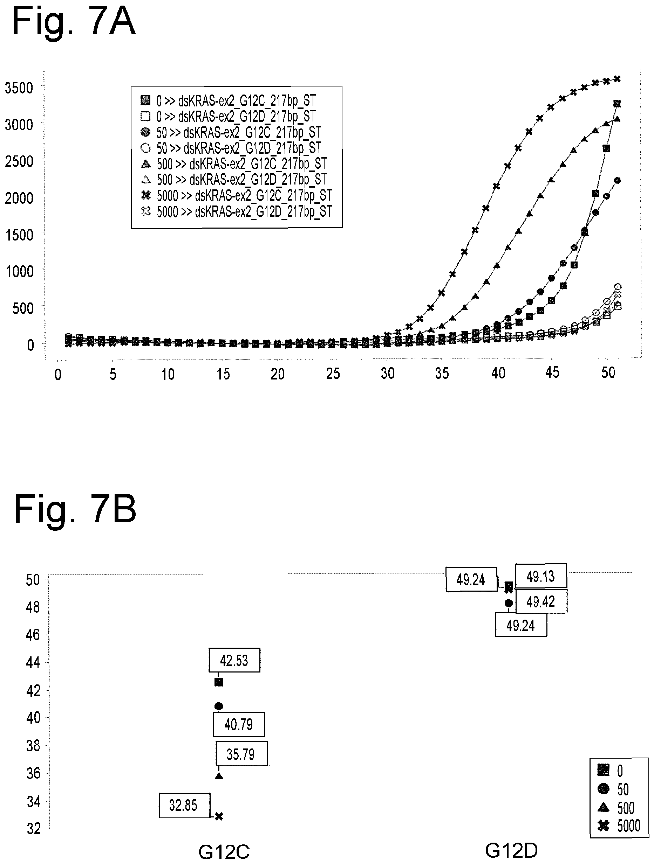

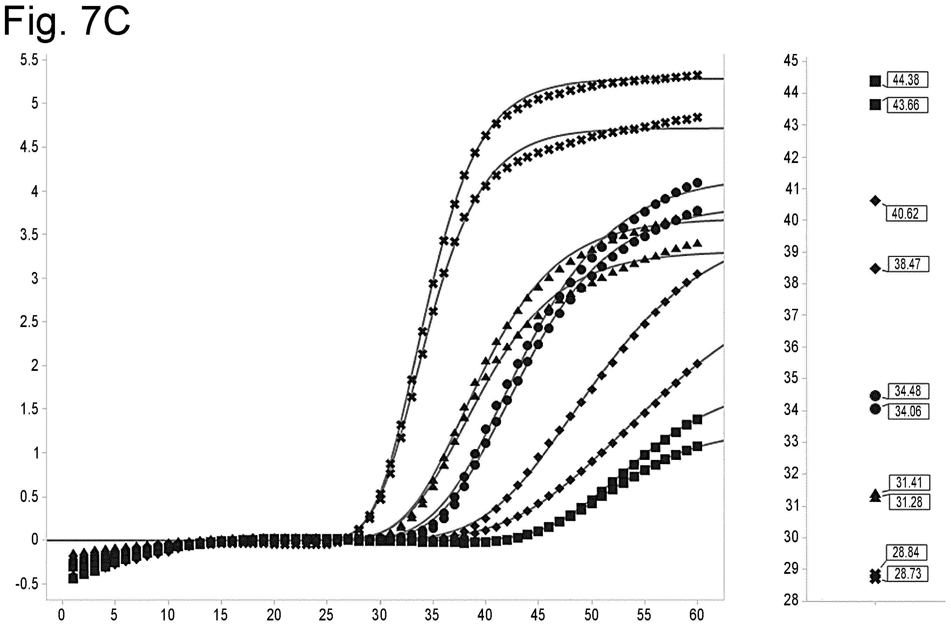

**Figure 7****:** shows the demonstration of discriminating between KRAS G12C from KRAS G12D and wild-type background in accordance with the concept shown in Figure 6 in a multiplex (*i.e.* with an oligonucleotide pool containing primers that are needed to amplify multiple targets, as well as mediator probes). An ARMS primer with a stem loop that enables selective amplification of KRAS G12C is added together with a reverse primer, as well as a mediator probe that is designed to bind to the stem loop proportion of the incorporated KRAS G12C ARMS primer. The mix also contains 10,000 genomic copies of wild-type DNA, along with the polymerase and all other components that are needed to have a functional PCR reaction. Either the synthetic KRAS G12C mutant target (see top panel A, and left part of bottom panel B) or the synthetic KRAS G12D mutant target (see top panel A, and right part of bottom panel B) is added in a 10-fold titration series (with estimated input 5000-500-50-0 copies/PCR) to the mix). The mix is added to different wells of a 96-well qPCR compatible plate. The top part represents the PCR curves, the bottom part shows the Cq values of the same PCR curves. The observed data demonstrate that the stem loop in the ARMS primer enables the discrimination of KRAS G12C from KRAS G12D and wild-type background down to 500 copies and lower in 10,000 genomic copies of wild-type DNA; panel C shows the performance of the same ARMS primer with a stem loop for KRAS G12C in an integrated sample-to-result instrument in presence of formalin-fixed paraffin-embedded (FFPE) wild-type sample that was upfront quantified to contain about 10,000 copies of genomic DNA per PCR chamber and various copy numbers of synthetic mutant target as indicated.

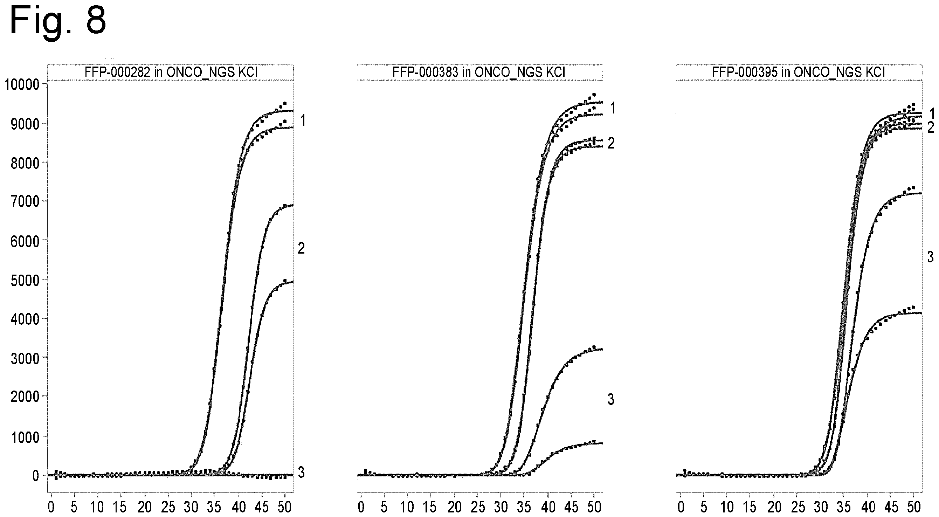

**Figure 8****:** shows how the degree of fragmentation can be verified using KIF11-based QC plex. The righthand panel shows a good quality sample; in this case, all curves (1, 2, & 3) cross the threshold around the same Cq value. In the middle panel, the curves for the middle (2) and longest (3) amplicon shift to the right (*i.e.,* in the direction of higher Cq values). This means that the sample contains less "long" DNA and is therefore fragmented. The left-hand panel represents a very fragmented sample, as can be appreciated by the absence of a qPCR result for the longest amplicon (3) and the shift to higher Cq values for the middle amplicon (2).

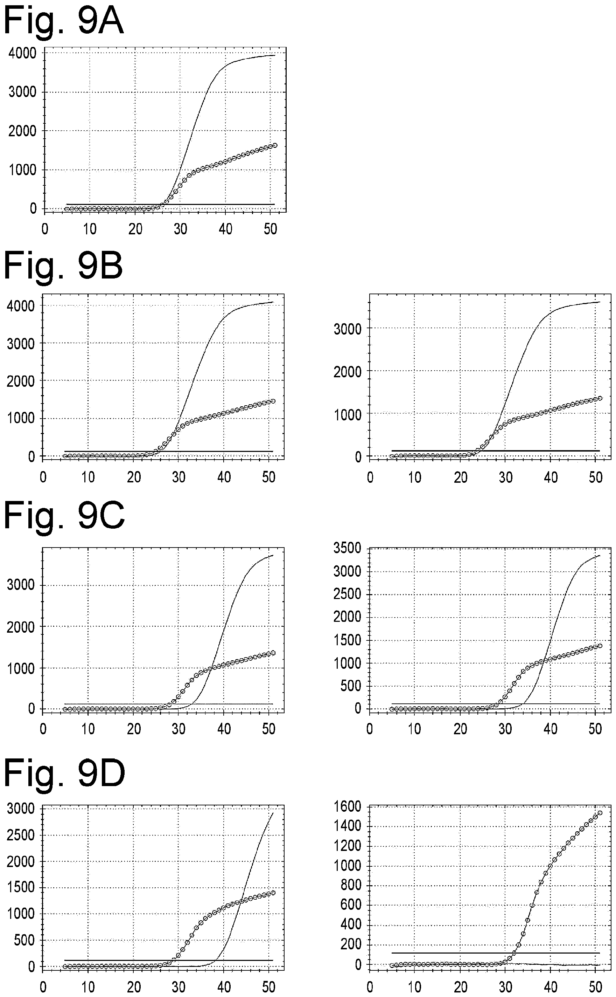

**Figure 9****:** shows that the delta Cq between the long and short KIF11 amplicons as detected by mediator chemistry strongly correlates with the degree of DNA fragmentation in the evaluated FFPE samples. The fitted curves for the short KIF11 amplicon are indicated with lines with circles, while the fitted curves for the long KIF11 amplicon are indicated with regular lines. The panels A-D show as follows: (A) No fragmentation, high quality gDNA sample, Delta Cq (long-short): 0.2; (B) Low fragmentation samples; Delta Cq (long-short) 1.2 (left) and 0.9 (right); (C) Medium fragmentation samples; Delta Cq (long-short) 4.7 (left) and 5.6 (right); (D) High fragmentation samples; Delta Cq (long-short) 9.4 (left) and N/A (long amplicon not detected; right).

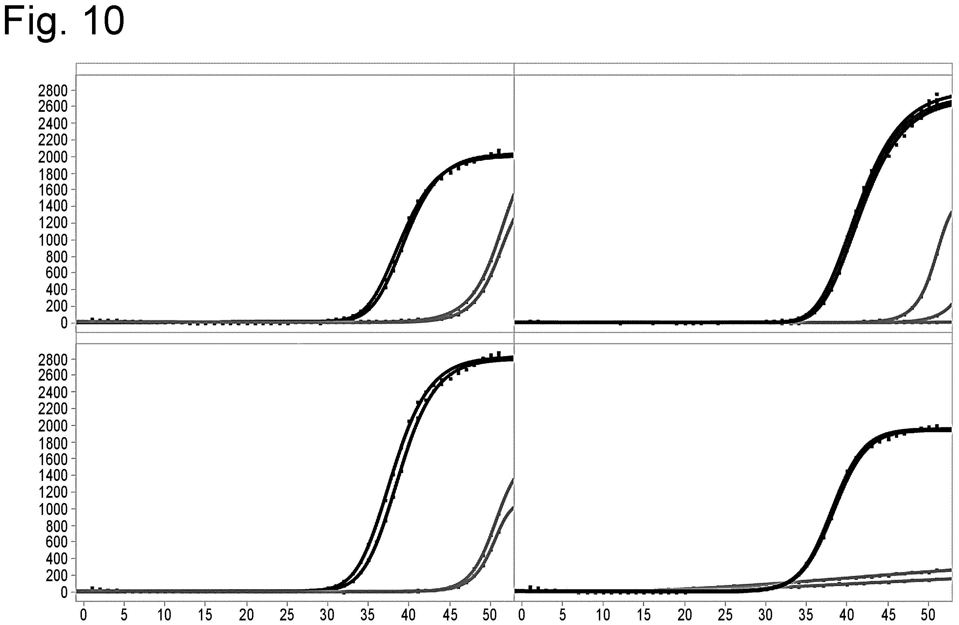

**Figure 10****:** mutation detection using FuseTag primers in a singleplex PCR in a 96-well format qPCR instrument for four different targets. Top left: BRAF V600E; Top right: EGFR E709K; Bottom left: EGFR S768I; Bottom right: EGFR L861Q; The X-axis indicates the number of PCR cycles; The Y-axis indicates the fluorescence (arbitrary units). The targets are added as synthetic mutant targets at 200 copies in the PCR reaction (which always contains 2,000 copies of genomic wild-type DNA, as well as the universal reporters, and the polymerase, dNTPs and PCR salts). Black lines represent the amplification results of 200 copies in a gDNA background, grey lines where genomic wild-type DNA is present, but no synthetic mutant targets are added.

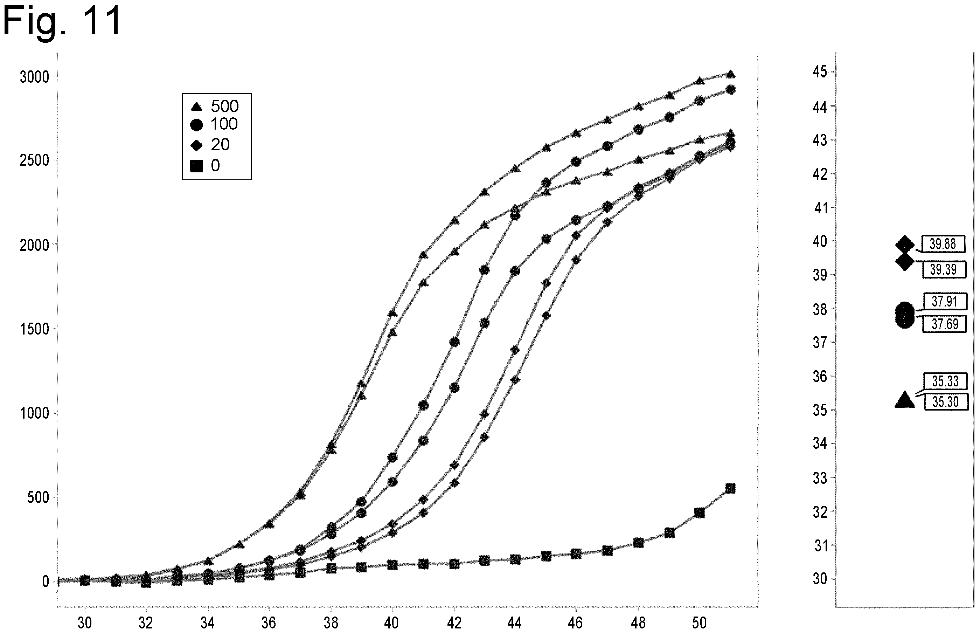

**Figure 11****:** mutation detection using FuseTag primers in a multiplex PCR in a 96-well format qPCR instrument in a gDNA background. The targets are added as synthetic mutant targets at various concentrations together with the oligo pool containing primers and mediator probes, and 1000 copies of genomic wild-type DNA. The generic reporters, polymerase, dNTPs and PCR salts (e.g. MgCl₂) are added to each reaction together with a liquefaction buffer containing components that would be required to release DNA from a FFPE sample. Triangles indicate 500 copies of BRAF V600E target; filled circles 100 copies of target; diamonds 20 copies of target. Squares indicate negative control, i.e. 0 copies of target. The X-axis indicates the number of PCR cycles; The Y-axis indicates the fluorescence (arbitrary units).

### DETAILED DESCRIPTION

Present disclosure provides methods, kits, kits of parts, systems, and components thereof, for performing multiplex detection of genetic targets using customized genetic target panels in a generic detection cartridge.

It was shown that the system, or parts of a system, methods, kits, kits of parts, and components thereof, can be used to detect the presence of one or more target sequences using generic (*i.e.* not target-specific, and hence can be purchased at high volume and hence cheap) fluorescently labelled reporters in a multiplex that can exceed the number of fluorescence signals that can be discriminated in a single PCR reaction chamber, in a device that requires only a single sample inlet source. In contrast, the contemporaneous technology is limited by the number of fluorescence signals that can be discriminated in a single PCR reaction (e.g. 6 signals can be discriminated in the case of Idylla^{™} with conventional technology, since there is no differentiation of what goes into each of the different PCR chambers (there is only 1 sample inlet)). With the present technology, generic fluorescently labelled reporters can be used while at the same time the multiplex capabilities across all PCR reaction chambers that are available in a device can be exploited.

As used herein, the term "genetic target" (which is used interchangeably herein with "target nucleic acid", unless the context requires otherwise) refers to any gene, transcript, nucleic acid in general, or fragment or forms of any of the above, which can be targeted for detection or investigation by a diagnostic assay. Examples of genetic targets include, but are not limited to genes, sometimes referred to "target genes", gene mutants, particular mutations or short nucleotide polymorphisms (SNPs) within genes, allelic forms, or genetic variants. As used herein, the term "variant" may refer to any genetic variant, *i.e.* any genetic feature that is known or expected to be different across genetic samples. The term "variant" as used herein can be interpreted as a type of a "genetic target" and can refer to particular mutations, SNPs, or genetic rearrangements, including duplications and deletions. Genetic rearrangements, duplications and deletions may affect small regions, such as regions of one or a few basepairs (bp), or large regions, such as large chromosomal defects stretching over multiple kilobasepairs (kbp). In the present context, the term "variant" will typically refer to a known genetic difference between a tissue that requires monitoring in a subject and a normal tissue and can be treated as a term synonymous to the terms "specific allele", "mutation", "SNP", "variants" in line with their standard meaning as used in the field of molecular biology and biotechnology. When reference is made herein to a member of a primer pair being allele-specific, the said member will bind the specific allele but not any other allele of a gene under appropriate conditions. Similarly, when reference is made herein to an allele-specific probe, the said probe will bind and/or detect the specific allele of a gene but not any other allele under appropriate conditions.

As used herein, the term "oligonucleotide" relates to a relatively short or oligomeric, usually below 200 nucleotides ("nts"), nucleic acid. Oligonucleotides are frequently synthetic and can comprise various modifications, like modified bases, or be conjugated to various molecules of different functionalities, etc. As used herein, the term "oligonucleotide subset" is to be interpreted as a functionally-linked group of oligonucleotides that are specific to a "genetic target". The oligonucleotides in an oligonucleotide subset will normally hybridize, depending on the application, within or around the sequence covering or flanking the genetic target, possibly to enable the genetic target's amplification or detection. A typical target-specific oligonucleotide subset will include at least one primer, likely a primer pair, and possibly also an oligonucleotide probe specific to a genetic target such as a genetic variant or a mediator probe. In general, the term "oligonucleotide mixture" will be used herein to describe a target-specific amplification primer pair ("oligonucleotide subset") and a probe, preferably a target-specific probe, even more preferably a mediator probe. As used herein, the term "multiple" in "multiple oligonucleotide subsets" or "multiple genetic targets" or "multiple oligonucleotide mixtures" is to be understood as referring to more than one, e.g. a plurality of genetic targets. In the context of a multiplex amplification or a multiplex PCR, the term "multiple" will usually refer to more than 1, such as, 2, 3, 4, 5, 6, 7, 8, 9, 10 or in the range of multiples of 10. Then, the term "mix of multiple oligonucleotide subsets" is to be interpreted as a composition, possibly a solution or at least a partially dried form thereof (e.g. lyophilized) containing the multiple oligonucleotide subsets mixed together. In certain contexts, the term "mix" can also refer a "panel" or a "set" comprising the multiple oligonucleotide subsets.

The term "nucleic acid" and its equivalent "polynucleotide", as used herein is given the regular meaning in the field and refers to a polymer of primarily ribonucleotides or primarily deoxyribonucleotides bound together by phosphodiester linkages between the nucleotide monomers. (Deoxy)nucleotides are phosphorylated forms of (deoxy)nucleosides, which most commonly include adenosine, guanosine, cytidine, thymidine, or uridine. These nucleosides consist of a pentose sugar, being ribose or deoxyribose, and a nitrogenous base ("nucleobase", or simply, "base") being either adenine, guanine (that are purines), cytosine, thymine, or uracil (being pyrimidines). The sequence at which these bases (or their nucleosides, or the nucleotides of the latter) follow in a nucleic acid strand is termed "nucleic acid sequence" and is conventionally given in a so called 5'-end to 3'-end direction referring to chemical orientation of the nucleic acid stand. The "5'" originates from the reference to the 5' carbon of the first (deoxy)ribose ring from which the reading of the nucleic acid sequence begins, and the "3'" originates from the 3' carbon of the last (deoxy)ribose ring on which the reading of the nucleic acids sequence ends. A nucleic acid sequences can e.g. be ATATGCC, which is to be interpreted herein as referring to 5'- ATATGCC - 3' nucleic acid sequence. Under the same convention, the latter sequence will be complementary to the sequence 5' - GGCATAT-3', or simply GGCATAT. Nucleic acids include but are not limited to DNA and RNA, including genomic DNA, mitochondrial DNA or methylated DNA, cDNA, mRNA, rRNA, tRNA, hnRNA, microRNA, IncRNA, siRNA, and various modified versions thereof. Nucleic acids can most commonly be obtained from natural sources like biological samples obtained from different types of organisms. On the other hand, nucleic acids can also be synthesized, recombined, or otherwise produced in any of the known human-devised methods (e.g. PCR)

As used herein, the term "separately" in the particular contexts of the mix of multiple oligonucleotide subsets being *provided separately* from the cartridge, or the cartridge being *provided separately* from said mix, is to be understood that there is no physical connection present between the mix and the cartridge until the moment a user inserts the mix or a part thereof into the cartridge. For example, the mix can be provided in a liquid form inside of a vial or a tube or any other container. In such instance, a user would open such container and pour or, more likely transfer by means of an e.g. pipette, its contents comprising the mix into the cartridge. Alternatively, the mix can be spotted and/or absorbed onto a solid medium, such as cellulose or a piece of parchment, and provided into the cartridge while bound onto said medium. Other alternatives also exist, including beads, dissolvable tablets, or capsules, etc. In any of these events, the mix in the context of the present invention is not physically comprised inside of the cartridge until being transferred thereto by a user before, together, or after also providing into the cartridge the biological material or isolated nucleic acid. In possible instances, the mix and the cartridge can even be provided at different points in time, e.g. when they are sold or shipped on different days to the user.

As used herein, the term "biological sample", or simply "sample", is intended to include a variety of specimen or solutions of biological sources, which contain nucleic acid and/or cellular material, irrespective whether it is freshly obtained from an organism (*i.e.* fresh tissue sample) or preserved by any method known in the art (e.g. an frozen or an FFPE sample). Examples of biological samples include: cultures of cells such as mammalian cells but also of eukaryotic microorganisms, body fluids, body fluid precipitates, lavage specimen, fine needle aspirates, biopsy samples, tissue samples, cancer cells, other types of cells obtained from a patient, cells from a tissue or *in vitro* cultured cells from an individual being tested and/or treated for disease or infection, or forensic samples. Non-limiting examples of body fluid samples include whole blood, bone marrow, cerebrospinal fluid (CSF), peritoneal fluid, pleural fluid, lymph fluid, serum, plasma, urine, chyle, stool, ejaculate, sputum, nipple aspirate, saliva, swabs specimen, wash or lavage fluid and/or brush specimens. In an aspect, the sample is a mitochondrial DNA, cDNA, mRNA, rRNA, tRNA, hnRNA, microRNA, IncRNA, cfDNA, cell-free tumor DNA or siRNA sample.

As used herein, the term "liquid biopsy" or a "liquid biopsy sample" shall be understood as referring to any non-tissue specimen, especially body fluid sample, obtained from a subject. Liquid biopsy sources include but are not limited to blood, plasma, serum, urine, cerebrospinal (CSF) fluid, amniotic fluid, other body fluids such as saliva, sweat, tears, breast milk, semen, stool, pleural fluid, peritoneal fluid or washings, etc. Analyzing nucleic acids in liquid biopsy samples can minimize the need for expensive, invasive, and frequently painful tissue and/or tumor biopsies to enable dynamic disease or other physiological state monitoring. For example, in cancer patients, cell-free tumor DNA or RNA extracted from liquid biopsies can potentially be used in detection of mutations, translocations or copy number alterations, and the expression of specific cancer markers. Blood (plasma, serum or whole blood alike) is the most commonly described fluid used in liquid biopsy sample analysis in humans. In cancer patients, blood is the source of circulating tumor cells (CTCs), and cell-free DNA (cfDNA) and cell-free RNA (cfRNA) including circulating tumor DNA (ctDNA) and circulating tumor RNA (ctRNA), respectively, released by tumor tissues, which can be used to detect mutations present in the patients' tumors. The DNA can be methylated or not methylated. Of note, ctDNA comprises however only a tiny fraction of cfDNA present in the blood, which highlights the importance of maximizing sample volumes for nucleic acid analyses in order to detect rare mutations. Furthermore, cfDNA is always of low quality and fragmented to the approximate size of a nucleosome (140 bp to 170 bp). Consequently, for certain cancer types including kidney, prostate, and upper and lower tract urothelial carcinomas, alternative liquid biopsy approaches such as urine may be a richer source of tumor-derived material. Urine also has other unique benefits such as ease of acquisition (does not require trained medical staff), lack of patient discomfort (increased patient compliance), and may have fewer contaminating proteins compared to blood.

Also, as used herein the term "nucleic acid isolation" is to be interpreted as any form of releasing nucleic acids from a biological material to make it available for amplification. Within the ambit of present disclosure, the term can encompass any procedure involving liquefaction of a biological sample or any nucleic acid extraction or purification on a solid support, such as silica.

The term "polymerase chain reaction" or "PCR" is to be understood as referring to the common laboratory nucleic acid amplification technique relying on thermal cycling and the use of at least a primer, typically primer pair, and a DNA polymerase. "Quantitative PCR" or simply "qPCR" is herein given the definition of a laboratory technique based on PCR, which is used to amplify and possibly simultaneously detect or quantify a targeted DNA molecule. In contrast to standard PCR where the product of the reaction is detected at its end, *i.e.* after thermocycling has finished, the key feature of qPCR is that the DNA product is being detected during thermocycling as the reaction progresses in "real time"; hence, the alternative name of qPCR *"real-time PCR".* There currently exist many different types of qPCRs. For example, when starting with a reverse transcription (RT) step, qPCR can be used to quantify numbers of messenger RNAs and is then called a reverse transcriptase qPCR or an RT-qPCR. As used herein the terms *"quantitative PCR"* or simply *"qPCR"* will be employed with preference over the term *"real-time PCR"* or *"RT-PCR"* in order to avoid confusion with *reverse transcription PCR,* also frequently abbreviated as RT-PCR. Most qPCRs use one of the following most common methods for detecting the product amplification in real-time involving fluorescence: (a) intercalation of non-specific fluorescent dyes with any double-stranded DNA, (b) the fluorescence is generated by a nucleic acid binding fluorochrome upon binding to double-stranded DNA, (c) a fluorophore is released by digestion of a probe during elongation of the primers or (d) by a fluorochrome bound to a probe that fluoresces after binding to the target during nucleic acid synthesis. The fluorescence emitted from the reaction mixtures is monitored in real-time as the amplification reactions occur, but in the initial amplification cycles the fluorescence is too low to be distinguishable from the background. The fluorescent signals generated during thermocycling are detected by an appropriate optical detection system and tracked from the moment they pass the background threshold till the reaction reaches plateau. The copy number of the target sequences can be estimated using either relative or absolute quantification strategy, typically by analyzing the shape of the obtained amplification curve (standard curve strategy), comparison to a standard reference or by determining when the signal rises above some threshold value (often called the Ct value, but sometimes also Cp value or Cq value). In relative quantification, the target nucleic acid levels estimated in a given sample using the Ct or standard curve analysis are expressed as relative to values obtained for the same target in another reference sample, for example, an untreated control sample. Conversely, in absolute quantification the qPCR signal is related to input copy number using a standard curve or can also be calculated according to a more recent digital PCR method. For the moment being, the first strategy is still more prevalent and bases the estimation of the target DNA amount by comparing the obtained values with a previously made standard curve. These and other qPCR quantification strategies are broadly known in the art and their calculation can differ in smaller or greater depending on a given application and a qPCR system.

Although fluorescence is by far the most commonly used method, any measurable property can be used in qPCR.

As used herein, the "quantification cycle" or "Cq" value of an amplification reaction is defined as the fractional number of cycles that are needed for the fluorescence to reach a threshold value, indicating the position of the amplification curve with respect to the cycle axis. Because Cq is directly related to the starting concentration of the target, and the difference in Cq values is related to the starting concentration ratio, Cq values are inverse to the amount of target nucleic acid that is in the sample, and correlate to the number of target copies in the sample. Lower Cq values (typically below 29 cycles) indicate high amounts of the target nucleic acid. Higher Cq values (above 38 cycles) mean lower amounts of target nucleic acid.

A ΔCq is calculated in various methods, kits, kits of parts, systems, or components disclosed herein, which is a log-ratio of the concentrations, *i.e.* the log of the concentration of the target nucleic acid, normalized to the concentration of the reference nucleic acid, such as, for instance, KIF11 gene or a region thereof. In some embodiments, ΔCq is calculated between the threshold cycle numbers (Cq) of first and second, and potentially third KIF11 amplification reactions as a measure for the presence of genomic DNA (integrity or contamination).

It was established that KIF11 amplicons are exceptionally useful in the double ΔCq analysis of qPCR results developed by Livak and Schmittgen (2001 Methods 25:402-8) or the analysis based on the standard curve method for relative quantification as developed by Pfaffl (2004 Quantification strategies in real-time PCR. In M. W. Pfaffl, A-Z of quantitative PCR. La Jolla, CA, USA: International University Line).

In general, the Cq results of qPCR measurements are foremost dependent on the starting concentration of the target nucleic acid, especially in the same experiment. Accordingly, qPCR results can be reported as ΔCq and double ΔCq values, which represent the gene expression ratio and fold change between experiments, respectively. Hence, a ΔCq = 0 indicates that the starting concentration of target nucleic acid and the reference gene are the same. The value of ΔCq and double ΔCq to consider an experiment meeting a preset requirement, such as for instance integrity of nucleic acids, contamination by gDNA, gDNA fragmentation, process control, etc., can be established by the skilled artisan according to the specific needs.

Quantification of output signals, such as signals produced by a detectable probe, e.g. fluorescence, in an amplification reaction, relates to the process of mapping input values from a large set to output values in a smaller set, often with a finite number of elements, such as e.g. rounding and truncation, and can be performed by any means known to the skilled artisan, preferably via digital signal processing using dedicated software.

Normalization refers to the process of adjusting values measured on different scales, such as the signals produced by a target nucleic acid in an amplification reaction, to a notionally common scale, such as the signals obtained with a reference probe, e.g. KIF11, in an amplification reaction. For instance, a value obtained with a target nucleic acid in an amplification reaction is adjusted to the value of a KIF11 gene amplification reaction, preferably the target nucleic acid and the KIF11 gene are amplified in the same amplification reaction and/or obtained from the same sample.

The threshold value represents the number of amplification cycles or elapsed time of amplification required for a detectable signal, such as a fluorescent signal, to exceed the basal threshold level ("background noise"), indicative for a positive PCR result. A threshold value may be a threshold cycle number in a thermal cycling amplification reaction, or the threshold value may be a time value (e.g., an elapsed time of amplification) in an isothermal nucleic acid amplification reaction. A threshold value can be determined by any means known to the person skilled in the art. In order to perform quantitative PCR, a threshold cycle value is determined for each target nucleic acid being amplified in the test and calibration samples. It is important that the method used to determine threshold values give reproducible values. By locating the threshold value in the log phase of the growth curve, such reproducibility is achievable. Preferably, threshold values are derived from first or second order derivatives of the growth curve.

The threshold value can be determined by calculating the cycle number or time value associated with the positive peak of the first derivative of the growth curve. The threshold value (e.g., the threshold cycle number in thermal cycling amplification or time value in isothermal amplification) can also be represented by the location of the peak of the second order curve. A threshold value for can be determined for a nucleic acid sequence amplification by: (i) deriving a growth curve for the nucleic acid sequence from the measured signals; (ii) calculating a derivative of the growth curve; (iii) identifying a characteristic of the derivative e.g. the first and/or second derivative; and (iv) determining the threshold value associated with the characteristic of the derivative.

As used herein, the term "primer" refers to an oligonucleotide, whether occurring naturally as in for example a purified restriction digest or produced synthetically, which is capable of acting as a point of initiation of nucleic acid sequence synthesis when placed under conditions in which synthesis of a primer extension product which is complementary to a nucleic acid strand is induced, *i.e.* in the presence of different nucleotide triphosphates and a polymerase in an appropriate buffer ("buffer" includes pH, ionic strength, cofactors, etc.) and at a suitable temperature. One or more of the nucleotides of the primer can be modified, for instance, by addition of a methyl group, a biotin or digoxigenin moiety, a fluorescent tag or by using radioactive nucleotides or universal detectable marker, in which case the primer can act as a probe. As used herein the terms "variant-specific primer" or "allele-specific primer", which are used interchangeably, refer to a primer that specifically binds to a variant sequence. Analogously, the term "variant-specific primer pair" refers to a primer pair that, in a PCR reaction, is intended to produce an amplicon only if the variant is present. A variant-specific primer pair may e.g. include a variant-specific primer, such that amplicons are only generated if the variant is present and the variant-specific primer has a suitable binding place. Alternatively, a variant-specific primer pair may include two primers which only bind in the correct orientation and at a suitable distance if the variant is present. For example, if the variant is a deletion, in the absence of the deletion the distance between the primers of the primer pair may be too large to generate an amplicon in the PCR reaction. In the presence of the deletion, the target-bound primers of the variant-specific primer pair have a correct distance for amplicon generation. The same concept can be applied to gene rearrangements. A type of a variant-specific primer is a so called "ARMS-primer". ARMS stands for Amplification Refractory Mutation System, which is a frequent application of PCR for identification of point mutations or polymorphisms, in which DNA is amplified by allele specific primers. The ARMS PCR uses a pair of primers, including an ARMS primer and usually a common PCR primer. The ARMS primer will normally have the following spatial features: (1.) length of usually about 20 to 40 bp; (2.) The nucleotide at the 3' end of the primer is usually complementary to the target nucleotide, *i.e.* G for C or C for G and T for A or A for T. Mismatch at this position can dramatically reduce the amplification. A:G, G:A, and C:C mismatches have the worst effect whereas the other mismatches have varying degrees of effect. For example in a mutation with A-T substitution the ARMS primer for the mutant allele should have the last nucleotide complementary to the nucleotide T, *i.e.* it should have A. The primer for the normal allele at the same position should be complementary to the nucleotide A, *i.e.* it should have T; (3.) possibly, an additional one or more mismatches at one of the last five to ten nucleotides of the ARMS primer to further increase its specificity. In some embodiments, allele-specific primers, such as ARMS primers comprise a stem-loop structure when hybridized to the target nucleic acid sequence (*see e.g.,* Figure 6). The term "amplicon" refers to the result of producing one or more copies of a genetic fragment or target sequence (amplification of a genetic fragment or target sequence) , which can be formed by any means known to the person skilled in the art, such as by PCR. In this context, an amplification reaction refers to the production of one or more copies of a genetic target or target nucleic acid. As used herein, the term amplicon encompasses the term "PCR product."

The disclosed methods, kits, kits of parts, systems, or components, relate to a cartridge for an automated system, possibly a PoC system or device. As used herein, the term "cartridge" is to be understood as a self-contained assembly of chambers and/or channels, which is formed as a single object that can be transferred or moved as one fitting inside or outside of a larger instrument that is suitable for accepting or connecting to such cartridge. A cartridge and its instrument can be seen as forming an automated system, further referred to as an automated platform. In some embodiments, the system further comprises one or more reaction components, such as oligonucleotide mixtures, reporter molecules, and reagents for amplification reactions, such as buffers, salts, enzymes, etc., ("PCR mix"). Some parts contained in the cartridge may be firmly connected whereas others may be flexibly connected and movable with respect to other components of the cartridge. Analogously, as used herein the term "fluidic cartridge" shall be understood as a cartridge including at least one chamber or channel suitable for treating, processing, discharging, or analyzing a liquid, preferably a fluid. An example of such cartridge is given in WO2007004103. Advantageously, a fluidic cartridge can be a microfluidic cartridge. In general, as used herein the terms "fluidic" or sometimes "microfluidic" refers to systems and arrangements dealing with the behavior, control, and manipulation of fluids that are geometrically constrained to a small, typically sub-millimeter-scale in at least one or two dimensions (e.g. width and height or a channel). Such small-volume fluids are moved, mixed, separated or otherwise processed at micro scale requiring small size and low energy consumption. Microfluidic systems include structures such as micro pneumatic systems (pressure sources, liquid pumps, micro valves, etc.) and microfluidic structures for the handling of micro, nano- and picoliter volumes (microfluidic channels, etc.). Exemplary and very suitable in the present context fluidic systems were described in EP1896180, EP1904234, and EP2419705. In line with the above, the term "chamber" is to be understood as any functionally defined compartment of any geometrical shape within a fluidic or microfluidic assembly, defined by at least one wall and comprising the means necessary for performing the function which is attributed to this compartment. Along these lines, "amplification chamber" is to be understood as a compartment within a (micro)fluidic assembly, which suitable for performing and purposefully provided in said assembly in order to perform amplification of nucleic acids. Examples of an amplification chamber include a PCR chamber and a qPCR chamber. In accordance with the above, in alternative embodiments, such cartridges may comprise oligonucleotide generic probes. The terms "chamber" and "compartment", including the plural versions, are used interchangeably herein, unless the context requires otherwise.

The term "probe" relates in general to any measurable property of the said probe, which changes when the probe interacts with the analyte, because of which the interactions between the probe and the analyte can be studied. The probe is preferably a nucleic acid which has a tag, e.g. by being labelled either radioactively or chemically, even more preferably fluorescently labelled. The measurable property of the target detectable probe will change when the target is amplified, e.g. producing a measurable signal, similarly the measurable property of the KIF11 detectable probe will change when KIF11 is amplified, e.g. producing a measurable signal. Preferably, the signal produced by the target detectable probe is different from the signal produced by the KIF11 detectable probe, allowing a differentiation of the signals. As used herein, the term "generic reporter", which is herein used interchangeably with the term "generic reporter molecule", is to be interpreted as any oligonucleotide probe capable of generating a detectable signal or a change in signal, as a result of its hybridization with a at least partially, preferably substantially, complementary to at least its part, the unique generic sequence tag. In a simplification, a generic reporter can be interpreted as a labelled probe, specific to a generic sequence tag. In some embodiments, a generic reporter comprises a singled-stranded DNA molecule, comprising the following elements: a first member of a fluorophore/quencher pair; a stem-loop structure; a second member of the fluorophore/quencher pair; a UGST binding site, which is complementary to the UGST of a mediator probe; a polymerase extension blocker; wherein the members of the fluorophore/quencher pair are positioned, via the stem-loop, to quench the fluorophore in the absence of binding and extension of the UGST of the mediator probe. Exemplary, non-limiting generic reporter molecules are illustrated in Figures 2 and 6.

As used herein, the term "mediator probe" refers to a single-stranded DNA sequence comprising the following elements, from 5' to 3': a first portion, the first portion comprising a unique generic sequence tag ("UGST"); a second portion, the second portion comprising a sequence that is complementary to a first strand of the target nucleic acid, or that is complementary to a portion of an allele-specific primer (or the complement of a portion of an allele-specific primer). By way of example, in some embodiments, an allele-specific primer comprises a stem-loop structure when hybridized to its target. Upon amplification, the stem-loop sequence of the primer becomes part of the amplified sequence(s) (*see e.g.,* Figure 6). In some embodiments, a UGST of a mediator probe is complementary to all or a portion of such a stem-loop sequence or its complement.

As used herein, the term "stem-loop" also known as a "hairpin" refers to intramolecular base pairing occurring in single-stranded nucleic acids, such as primers and probes, in particular when two regions of the same strand, usually complementary in nucleotide sequence when read in opposite directions, base-pair to form a double helix that ends in an unpaired loop. The structure is a stem-loop or hairpin loop.

The term "generic sequence tag" is to be understood as a sequence, usually within the length range of oligonucleotides, not present or possibly present at a low to negligible abundance in the genetic information of the organism from which the genetic targets are being detected. Examples of possible unique sequence tags include but are not limited to nullomers, scrambled synthetic sequences, sequence derived from different/phylogenetically distant organism, Unique Molecular Identifiers etc. In the context of the generic sequence tag being "unique" to (an oligonucleotide) subset (from the mix)" is to be understood that exactly one generic sequence tag corresponds to exactly one "oligonucleotide subset" that is specific to one "genetic target". As used herein, an "oligonucleotide subset" includes those oligonucleotides specific to a target, and include, without limitation one or more amplification primers, and one or more probes, such as mediator probes.

As used herein, the term "detectable nucleic acid product" refers to a product or a byproduct from an amplification reaction of a genetic target with the oligonucleotide subset specific to said target. The detectable nucleic acid product is to be understood as being detectable by the virtue of comprising a "unique generic sequence tag" ("UGST") that can be detected by a generic reporter, e.g. being a probe with a label. An example of a detectable nucleic acid product can be an amplicon incorporating the generic sequence tag sequence and generated with a primer or a primer pair forming part of a subset from the multiple oligonucleotide subset. An alternative detectable nucleic acid product can be cleaved or otherwise released part of an amplicon, a primer, or a probe, said released part incorporating in its sequence the generic sequence tag. A specific example of such detectable nucleic acid product is a first portion of a mediator probe being released by cleavage (e.g., such as by the 5' - 3' exonuclease activity of a polymerase) and comprising the generic sequence tag, such as a unique generic sequence tag.

In brief, a method is disclosed wherein a user not only inserts a biological sample to a cartridge, but also the separately provided mix of multiple custom target specific oligonucleotide subsets. This is in contrast to the procedure with the existing assay-specific cartridges, wherein the target-specific oligonucleotides are provided inside of the cartridge. In presently disclosed instance, however, in order to enable multiplex nucleic acid amplification in the cartridge, the mix of target-specific oligonucleotide subsets has to be provided into the cartridge by a user, equally as the sample potentially containing the genetic targets of interest as defined by the mix. In an instance where at least one detectable nucleic acid product is generated from said amplification, a signal is generated and can be detected from at least one of the plurality of generic reporters comprised within the cartridge.

The presented herein approach allows for very rapid custom assay design, whereby the genetic target panels can completely be defined by the customers. Achieving this flexibility to design a completely-client defined panel and port it into a standard panel-specific cartridge is simply not possible under the current production pipeline reality of diagnostic cartridge manufacturers. The huge efforts and costs invested into bringing a standard panel-specific cartridge into market for a relatively small number of uses by perhaps a single user, would simply not only be non-profitable but likely would not even bring the return of the investment costs involved. However, within the ambit of the disclosed concept, wherein the major investments are spent on a generic detection cartridge and the customized panel design can later be relatively easily streamlined, possibly with help of an machine learning-powered algorithm, personalized panels for monitoring oncology patients are becoming feasible to come into practice. For example, in an interesting example of the disclosed method, at least one, preferably more, of the multiple oligonucleotide subsets are specific to a genetic target that was identified in a Next Generation Sequencing (NGS) analysis previously performed on a sample from an individual from whom the biological sample was obtained and provided into the cartridge. In such instance, for example, a tumor sample from patient is first analyzed by NGS for determining the key tumor-associated lesions. Based on the result, a custom panel of target-specific oligonucleotide subsets comprising oligonucleotide reagents specific to the selected identified lesions and genes of interest can relatively quickly be designed and produced. From this moment on, the status of the selected lesions and genes can readily and cost-effectively be monitored using the generic detection cartridge, the custom designed panel, and a sample from said patient, for example blood or plasma. Like this, following a surgery or a course of treatment, the patient's tumor status and response is monitored and surveilled on molecular level for any possible instances requiring medical intervention or change of treatment, without the need of repeating the NGS analysis.

In a next example of the disclosed methods, kits, kits of parts, systems, and components, compatible with the previous one, the plurality of generic reporters is immobilized inside of the integrated fluidic cartridge, preferably by being immobilized inside of the one or more nucleic acid amplification compartments. Depending on the design of a given generic cartridge, the immobilization can be done by covalent bonding or affinity interactions as known in the art, which could be useful in generic cartridges based on monolithic or etched chip-like structures with channels under controlled liquid flow. Alternative option involves providing reagents in a matrix-containing spot solution, which can later be dried or freeze-dried to a glass state or similar, not only immobilizing the reagents but also protecting them and stabilizing their storage life. Upon contact with aqueous solutions, such dried matrix becomes hydrated and releases captured therein reagents. In line with this, in an example of the disclosed methods, systems, components, kits, kits of parts, the plurality of generic reporters are immobilized in a spot solution.

If such immobilization is made inside of one or more amplification chambers, other reagents can naturally be included in the spot mix together with the generic reporters, which can include e.g. dNTPs and/or enzymes like polymerases and reverse transcriptases. Depending on stability considerations, one or more spots with different reagents can be deposited in the compartment of choice.

In another possible example, compatible with any of the above examples of disclosed methods, kits, kits of parts, systems, and components, spot solutions are provided e.g. including methylation sensitive enzymes. Along these lines, a methylation sensitive restriction enzyme (MSRE) or a methylation dependent restriction enzyme (MDRE) can be added to the spot solution. When the mixture comprising the isolated DNA and the mix of multiple oligonucleotide subsets enters the PCR chamber, the MSRE/MDRE digest unmethylated/methylated recognition sites when incubated at a certain temperature (20-50°C, typically 30-37°C). In a next step, the mixture of DNA, target specific oligonucleotide subset, and the MSRE/MDRE is heated to temperature between 50-110°C, typically between 70-99°C to inactivate the MSRE/MDRE. At the same time, optionally, a hotstart PCR enzyme can be activated, followed by a typical qPCR cycling protocol. Using this approach, any methylation signature can be easily detected within the same generic detection cartridge.

Spotting a generic reporter and/or other reagents is simple and positively correlates with prolonged shelf life. Depending on the reagent type, different sport solution compositions, or means of immobilization can be used. The spotting or immobilization positions for reagents like MSRE/MDRE can also be made in different compartments or channels, depending on a given cartridge infrastructure, position of heaters etc. For example, they can be also provided upstream of the amplification chamber.

Choice of placing different reagents and running processes in given compartments in general will depend on the internal design of a particular generic cartridge. In a next possible example of the disclosed methods, kits, kits of parts, systems, and components, compatible with any of the previous ones, the multiplex nucleic acid amplification and the generation of the signal from the at least one of the plurality of generic reporters is performed inside of the one or more amplification compartments. Such arrangement allows to monitor the reaction and target detection in real time, as well as places the detectable nucleic acid products generated during the amplification in close vicinity of the generic reporters. Other arrangements using a flow cell and separating the amplification and signal detection can also alternatively be envisaged.

In another possible example of hereby disclosed methods, kits, kits of parts, systems, and components, the nucleic acids isolated from the biological sample and the mix of multiple oligonucleotide subsets can be moved inside of the integrated fluidic cartridge into at least two or more different amplification compartments. This is beneficial in at least two instances. One, if repetitions, like duplicates or triplicates of the same reaction are considered, for example for borderline detectable low copy number targets, Or, two, for automated systems having a defined or a fixed number of wavelength-specific detection channels associated with amplification chambers and adapted for capturing signals from the provided therein reporters. In the latter instance, by separating the nucleic acid and oligonucleotide pool mixture between more than two amplification compartments, one can detect more targets from the multiplex reactions by providing or spotting different generic reporters in different amplification compartments, even though the different generic reporters can be conjugated with a dye detectable in the same channel. Hence, in a possible example of this example, different generic reporters are provided in the different amplification compartments within the same cartridge.

Further in an example of the above example, the signal generated in the presence of the detectable nucleic acid product can be generated from a light-emitting dye and, possibly where the different amplification compartments comprising the different generic reporters comprise a set of the same or at least partially overlapping light-emitting dyes. Along these lines, if we imagine a generic detection cartridge having 3 amplification compartments, each compartment monitored in 4 different channels (e.g. red, yellow, green, and blue), the nucleic acid and oligonucleotide pool mixture would be separated between the 3 compartments. In each of the 3 compartments, the same multiplex amplification would take place and in each compartment 4 different generic reporters could signalize in 4 different channels (the red, yellow, green, and blue) the presence of a detectable nucleic acid product of the amplification. Given the fact that each group of the 4 different generic reporters per compartment can be specific to a different target from the multiplex, 12 different targets from the multiplex can be associated with 12 different detection event over the 3 amplification compartments, each compartment allowing detection in 4 channels. In a possible embodiment of the last two examples, for time-considerations the multiplex nucleic acid amplifications with the mix can be performed simultaneously in each of said two or more amplification compartments.

In another aspect, disclosed methods, kits, kits of parts, systems, and components, are provided wherein one or more of the multiple oligonucleotide subsets comprises at least a primer comprising the unique generic sequence tag, and the detectable nucleic acid product is an amplicon comprising said unique generic sequence tag. In this simple embodiment, the oligonucleotide subsets can merely comprise target-specific primer pairs, wherein one of the primers per such pair comprise, e.g. in a stem-loop-structure the generic sequence tag that is detectable by a generic reporter in the generic detection cartridge. In this instance, the detectable nucleic acid product is the amplicon itself as generated as part of the multiplex amplification.

In a completely different alternative embodiment of disclosed methods, kits, kits of parts, systems, and components, are provided wherein one or more of the multiple oligonucleotide subsets comprises at least one primer and at least one mediator probe, said mediator probe comprising the unique generic sequence tag, and wherein the detectable nucleic acid product is a cleaved free mediator comprising said unique generic sequence tag. This embodiment is based on mediator chemistry principle as disclosed in EP2776585 from Albert Ludwig University of Freiburg, and schematically shown in Figure 2. It has been evaluated to work very efficiently in prototype cartridges as described in the Examples section below. We have further modified the original principle by combining it with ARMS primers, which resulted in improved sensitivities. Hence, in some embodiments of the latter example, the at least one primer is an ARMS primer.

For a better discrimination of closely positioned mutations, certain target-specific ARMS primers were further modified to include a stem-loop structure, in which the stem can optionally be composed of the target sequence. We further modified the mediator probe in such a way that it at least partially overlaps with the stem-loop-modified ARMS primer and the concept is show in Fig.6. In top panel A, the 5' end of the ARMS primer comprises a sequence complementary to the target sequence, whereas in the bottom panel B, the 5' end of the ARMS primer terminates with the stem-loop-stem structure that serves as a generic tail tag, which has advantages for streamlined design of such primers. The 3' component of the stem of the ARMS primer can have a sequence that is derived from the target sequence, or can be another sequence. When the target is not amplified, the mediator probe cannot bind to the target sequence and hence cannot create a signal. The mediator probe also cannot bind to its complementary sequence within the ARMS primer, which is inside of the stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal. In line with the above, in specific embodiments of the last two examples, the ARMS primer comprises a stem-loop structure, and preferably, the mediator probe sequence further at least partially overlaps with the sequence comprised in said stem-loop structure.

In another example, a method is provided wherein the nucleic acid isolation from the biological sample is performed in the presence of the mix of multiple oligonucleotide subsets, possibly within the nucleic acid extraction compartment. In this particular instance, a user can add the mix of multiple oligonucleotide subsets together with the biological sample into the cartridge, which saves time. Surprising, we have observed it works very well with a nucleic acid isolation protocol comprising liquefaction protocol on the Idylla^{™}-based generic cartridge prototype. Alternatives comprise first providing the mix of multiple oligonucleotide subsets and having it pumped deepener into the internal cartridge space, followed by the biological sample addition once the mix achieves the desired compartment in the cartridge, and only them initiating the nucleic acid isolation protocol. Another alternative may involve including two entry ports within the cartridge, one for the mix and one for the biological sample.

In some examples, methods, uses, kits, kits of parts, systems, and components, are provided the biological sample is a solid tissue sample, possibly a fixed solid tissue sample, preferably a formalin-fixed paraffin-embedded (FFPE) sample. In such instances, the nucleic acid isolation could advantageously comprise or consist of liquefaction of said solid tissue sample.

In alternative examples, methods, uses, kits, kits of parts, systems, and components, are provided, wherein the biological sample is a liquid biopsy sample. In a particular embodiment of said example, methods, uses, kits, kits of parts, systems, and components, are provided wherein the nucleic acid isolation comprises isolation of cell-free nucleic acids. In a further embodiment of the latter embodiments, the nucleic acid isolation could advantageously comprise or consist of nucleic acid extraction, preferably on solid support.

In further examples, methods, uses, kits, kits of parts, systems, and components, are provided wherein at least a part of the oligonucleotide subsets from the mix of multiple oligonucleotide subsets, is designed by a computer-implemented method comprising machine learning or artificial intelligence.

Control is key for the robustness of any experiment and any decision based on such an experiment, especially when using sensitive techniques such as the amplification of target genes. However, it is necessary to keep in mind that successful amplification of a control gene only provides indirect information on the integrity of the target gene itself. Therefore, selection of appropriate control genes must be performed judiciously. At least one requirement is that control genes are not prone to variability, such as somatic mutations or copy number alterations, over a broad range of diseases, including cancers. Also, in a typical setting the effect of treatment options in various disease situations is assessed by following changes in target genes, necessitating normalization of data against a reference gene. Thus a pivotal prerequisite in the selection of reference genes is that they should not be affected by the treatment. To date, the most commonly used reference genes are housekeeping genes (HKG). However, normalization of data against random HKG may result in erroneous calculation of the normalization factor used to compare the treatment conditions, and therefore hiding biological differences among samples. Indeed, as long as the relation between the copy number, the stability of the reference gene, the target gene of interest and the disease is not known, selection of an appropriate reference gene is difficult. Herein we identified the Kinesin Family Member 11 gene ("KIF11"; NCBI Entrez Gene: 3832) to be highly genomically stable in terms of copy number alterations and somatic mutations in nearly all cancer types for which public data is available as well as in terms of SNPs. KIF11 was identified as a very promising generic control with a high likelihood of providing consistent performance across any global population.

As we identified KIF11 as a surprisingly stable genomic region among a majority of cancer types, the KIF11 gene or part thereof, such as a KIF11 region, can be used as an ubiquitously suitable genomic reference target (reference gene), as an alternative or in addition to contemporaneously used house-keeping reference genes, such as, for instance, described in Lemma et al. (Identification and Validation of Housekeeping Genes for Gene Expression Analysis of Cancer Stem Cells, PLOS ONE | DOl:10.1371/journal.pone.0149481 February 19, 2016), in a broad range of the methods, such as PCR, LCR, NGS, CGH. The use of such a ubiquitously suitable reference gene is especially useful in multi-gene panels, as it allows to use only a single reference gene when assessing the presence of mutations in the different genes, as opposed to using a stable region in each of the different genes to serve as the reference gene for assessing the presence of mutations in the corresponding gene, which would then require the use of more reagents and more spaces in the multiplex real estate of the test device.

In some examples, methods, uses, kits, kits of parts, systems, or components, are provided, wherein the mix of multiple oligonucleotide subsets comprises a subset specific to a region in the KIF11 gene. In their possible embodiments, said region in the KIF11 gene is used as a genomic reference gene, or alternatively, a KIF11 amplicon generated with said subset is used as a genomic reference gene. Preferred embodiments of present examples concern the human KIF11 gene, but possibly can also concern its other mammalian homologue. In particular examples, the region in KIF11 is located in any of the following exons 6, 8, 18, 21, intron 6, exon 21 - intron 21 boundary or the non-coding region of the KIF11 gene. In further embodiments, methods, uses, kits, kits of parts, systems, or components, are provided, wherein one or more of the multiple oligonucleotide subsets comprises a primer specific to a region in the human KIF11 gene, preferably wherein at least two of the multiple oligonucleotide subsets comprise primers specific to a different region in human KIF11 gene, wherein said primers are designed to generate two KIF11 amplicons of discernably different lengths. The term "KIF11 amplicon" as used herein, refers to an amplicon of the KIF gene or KIF11 region, wherein the amplicon is the result of an amplification reaction of the KIF11 gene or KIF11 region, respectively. The "KIF11 region" refers to a fragment or part of the KIF11 gene. The KIF11 gene refers to the Open Reading Frame and includes the 5' and 3' Untranslated Regions (UTR) as well as 5' and 3' located regulatory sequences. Accordingly, the term "non-coding region of the KIF11 gene" intends the 5' and 3' Untranslated Regions (UTR) as well as 5' and 3' located regulatory sequences.

As we identified KIF11 as a surprisingly stable genomic region among majority of cancer types, irrespectively of the disclosed herein methods, uses, kits, kits of parts, systems, or components, use of KIF11 as a genomic reference target or a house-keeping gene reference is hereby in general disclosed for any DNA or RNA amplification reaction, in particular in cancer. In particular examples, the region in KIF11 is located in any of the following exons 6, 8, 18, 21, intron 6, exon 21 - intron 21 boundary or the non-coding region of the KIF11 gene, and are hereby also disclosed as suitable genomic reference target regions.

In an embodiment, KIF11 or a KIF11 region can be used as a reference gene for assessing the integrity of gDNA. Genomic DNA (gDNA) integrity plays a critical role for the definition of gDNA quality and can influence downstream molecular applications, such as PCR, comparative genomic hybridization (CGH) or whole genome sequencing approach. Several factors affect gDNA integrity, mainly due to pre-analytical procedures such as sample DNA storage, repeated freeze-thawing, retention to the tubes, evaporation and/or denaturation. Additional factors such as humidity, temperature and variations in temperature, persistence of nucleases and other chemical agents as well as other suboptimal conditions that may occur during transportation and during gDNA extraction can also compromise gDNA integrity. The integrity of the gDNA is a determinant for the robustness and repeatability of experiments, especially for avoiding false negatives. High quality gDNA, such as gDNA of which the integrity is not compromised, refers to gDNA which is essentially pure, intact, double stranded, highly concentrated, and/or not contaminated, but which is at least suitable for the intended experimental procedure based on the gDNA. The methods provided herein using KIF11 are particularly suitable for determining gDNA fragmentation. As used herein, "absence of gDNA fragmentation" intends no detectable gDNA fragmentation with the methods of the present invention, including no gDNA fragmentation, *e.g.* intact gDNA.

The region in the KIF11 gene and the location of the members of the KIF11-specific primer pair (e.g. forward and reverse primers) to generate the KIF11 amplicon and the length of the KIF11 amplicon as used in the present invention can be determined according to the needs of the skilled artisan. For instance, a short KIF11 amplicon can be included as positive control if the amplicon length of the gDNA target(s) is about the length of this short KIF11 amplicon. If the amplicon length of the target(s) is variable, the skilled artisan may opt to include two KIF11 amplicons of discernable length, e.g. both a "short" and "long" KIF11 amplicon, such as amplicons of between 50-140 bases and of between 141-280 bases, respectively. Alternatively, the skilled artisan may opt to include two KIF11 amplicons of discernable length, wherein a first KIF11 amplicon is generated from a KIF11 region located entirely in a coding region of the KIF11 gene and a second amplicon is generated from a KIF11 region located in a non-coding region.

The person skilled in the art is fully knowledgeable designing appropriate primers and PCR conditions for generating KIF11 amplicons of the invention, such as KIF11 amplicons of discernably different lengths. Preferably, a KIF11 amplicon length between 50 and 280 bases, preferably 60 and 250 bases, such as 62 bases, 89 bass, 98 bases, 136 bases, 204 bases or even 280 bases is aimed for. When designing appropriate primers for generating KIF11 amplicons, the skilled person may consider the following. Preferably, stem loop secondary structures with low -ΔG values are avoided in the amplicon. Preferably, the amplicon is within a structurally stable section. Preferably, palindromic sequences are avoided in the amplicon. Preferably, G:C rich areas are avoided in the amplicon, e.g. approximately 50% G:C content is aimed for. Preferably, repetitive regions are avoided in the amplicon. Preferably, target regions over the intron-exon boundary or in non-coding regions are intended. It was surprisingly found that intron sequences, such as the intron 6 region as well as the non-coding sequence 3' of the KIF11 coding sequence were exceptionally stable. In possible embodiments, the region in the human KIF11 gene is located in any of the following exons 6, 8, 18, 21, exon 21 - intron 21 boundary, or intron 6 or non-coding sequence of KIF11 gene. Preferred subsets of forward and reverse primers are provided in Table 5.

When analysing cell-free DNA or cell-free tumor DNA, it is important to ensure that there is no gDNA contamination, especially if the assays in use do not discriminate between gDNA and cf/ctDNA sequences. To avoid contaminating gDNA, proper plasma sample preparation and storage is recommended. However, this sample preparation may not be 100% effective or prone to assay or operator errors. In order to quality control that there is no or only low levels of gDNA, it was found that determining the presence of KIF11 amplicons as described herein is very convenient for assessing absence of gDNA (which has a good integrity). Also, when analysing the level of expression of a certain target or by proxy determining the level of RNA, it is important to ensure that there is no gDNA contamination, especially if the assays in use do not discriminate between gDNA and cDNA sequences. To remove contaminating gDNA, enzymatic treatment of the samples with DNasel is recommended. However, this enzymatic treatment may not be 100% effective or prone to assay or operator errors. In order to quality control removal of gDNA, it was found that determining the presence of KIF11 amplicons as described herein is very convenient for assessing absence of gDNA.

In another aspect, kits, kit of parts, systems, or components thereof are disclosed comprising, provided as separate components:
- a mix of multiple oligonucleotide subsets, each of said subsets being specific to a genetic target and comprising a unique to said subset generic sequence tag,
   wherein each of said subsets is adapted to generate, under nucleic acid amplification conditions and in the presence of the genetic target, a detectable nucleic acid product comprising the unique generic sequence tag; and
- an integrated fluidic cartridge comprising:
   (i) an entry port for accepting a biological sample; possibly a second entry port for accepting the mix;
   (ii) a nucleic acid isolation compartment positioned downstream of the entry port, possibly said nucleic acid isolation compartment comprising or arranged to become fluidly connected with at least a reagent for nucleic acid isolation compartment, such as liquefaction buffer inside of the cartridge;
   (iii) reagents for nucleic acid amplification;
   (iv) one or more nucleic acid amplification compartments positioned downstream of the nucleic acid isolation compartment (e.g. nucleic acid extraction chamber), and
   (v) a plurality of generic reporters, wherein each of the plurality of generic reporters comprises a generic sequence specific to one of the unique generic sequence tags (that is unique to an oligonucleotide subset and is comprised in one of the detectable nucleic acid products) and is adapted to generate a signal in the presence of the detectable nucleic acid product comprising the unique generic sequence tag.

The polymerase extension blockers refers to an oligonucleotide that is made non-extendable by adding bases to the 3' end of the oligonucleotide (e.g. primer) that are not complementary to the target sequence and therefore do not base-pair and cannot be enzymatically extended and/or inhibit the polymerase during elongation without participating as a primer itself. Various polymerase extension blockers have been exemplified in the Examples section, such as, 3SpC3, but any polymerase extension blocker known in the art can be used, e.g. 3'-Spacer C3, 3'-Phosphat, 3'-ddC, 3'-Inverted End.

In some examples, kits, kit of parts, systems, or components thereof are provided wherein the plurality of generic reporters is immobilized inside of the cartridge.

In further examples, kits, kit of parts, systems, or components thereof are provided wherein an oligonucleotide subset from the mix of multiple oligonucleotide subsets comprises at least a primer and a mediator probe, and preferably wherein the primer is an ARMS primer. In some embodiments, further examples are provided wherein the ARMS primer comprises a stem-loop structure and wherein the mediator probe sequence at least partially overlaps with the sequence comprised in said stem-loop structure.

In some other examples, kits, kit of parts, systems, or components thereof, are provided wherein one or more of the multiple oligonucleotide subsets comprises a primer specific to a region in the human KIF11 gene, preferably wherein at least two of the multiple oligonucleotide subsets comprise primers specific to different regions in the human KIF11 gene, wherein said primers are designed to generate two KIF11 amplicons of discernably different lengths. In possible embodiments, the region in the human KIF11 gene is located in any of the following exons 6, 8, 18, 21, or exon 21 - intron 21 boundary.

Also disclosed are uses of disclosed methods, kits, kit of parts, systems, or components thereof, detecting multiple genetic targets, possibly in a sample from a cancer patient, possibly as part of post-NGS analysis patient surveillance or in minimal residual disease monitoring.

### EXAMPLES

### 1. Identification of a generically applicable genomic reference locus

As the goal of the present application was to provide for a generic platform capable of handling a great variety of custom-designed panels for very versatile and possibly genomically unstable diseases and targets, we have first attempted to identify a robust genomic reference locus or gene. In particular oncological diseases are frequently subject to genomic rearrangements and in our extensive oncological practice, we have tested many different house-keeping loci and believe that for each assay a reference gene or a group thereof is best selected in function of the tumor type. However, given the generic nature of the desired herein application, we have set out to screen for a locus that is minimally affected over a wide range of cancer types by different genetic variations, including point mutations and copy number changes. Our extensive literature and *in silico* analyses of our and others' data allowed us to top rank 100 potentially oncologically "stable" genes. These top ranked potential reference loci were further analyzed for somatic variations in The Cancer Genome Atlas (TCGA) database, queried via the public web portal https://www.cbioportal.org

The analysis allowed for further narrowing the selection to smaller number of targets, as a result of which we have identified and extensively verified that KIF11 appears to be an extremely promising genomic reference gene, very rarely affected by somatic mutations or copy number alterations in the tumor samples we tested or screened to date. The suitability of KIF11 as pan-cancer "stable" reference control could also be confirmed in a great variety of tumors of 228 studies included in the TCGA at the time of our analysis, with a slight exception of 2-7% of prostate cancers (TCGA reported amplifications or deletions, depending on the study) and 9% of nerve sheath tumors (amplifications). The latter analysis appears to confirm the surprising suitability of a previously unreported as a genomic reference gene KIF11 for being used as a generically stable control locus for a very wide variety of genomically unstable samples such as tumor samples. Our finding is even more surprising as KIF11 increased expression has widely been reported in many different cancer types and it has even been proposed to be an oncogene or a potential tumor marker in different cancer types (Daigo et al., 2018 Int J Oncol 52:155-165; Pei et al. 2017 Oncol Lett 6618-6626 https://doi.org/10.3892/ol.2017.7053; Imai et al. 2016 Pathobiology 84:16-24). Despite its reported transcriptional overexpression, based on our investigations and the TCGA-obtained data, KIF11 genomic locus appears as exceptionally stable and only when designing assays for prostate cancers or nerve sheath tumors, additional studies would be recommended to de-risk genomic stability of KIF11 as control region of choice.

To further corroborate our finding, we have extensively tested stability of selected KIF11 regions in a broad range of samples and online databases. In particular, for common genetic variations (SNPs), we particularly focused on intron 6, exons 6, 21, intron-exon boundaries around the latter, and the two longest exons 8 and 18 able to accommodate long amplicons (243 bp and 280bp, respectively).

In conclusion, KIF11 appears to be highly genomically stable in terms of copy number alterations and somatic mutations in nearly all cancer types for which public data is available, as well as, in terms of SNPs for the investigated exons and exon 21-intron 21 boundary. Consequently, KIF11 appears to be a very promising generic control with a high likelihood of providing consistent performance across any global population.

### 2. Selection of multiplex amplification chemistry with generic reporters

For the selection of a proof-of-principle nucleic acid amplification strategy comprising generic reporters, several approaches were evaluated. Examples include, but are not limited to, anisotropic loop hairpin primers as described in WO2020/165180 of Biocartis NV, a possible combination of PASS primers and MNAzyme technologies of SpeeDx Ltd as described, e.g., in EP2753717, EP2817421, and EP1948822, or a mediator probe chemistry as described in EP2776585 from Albert Ludwig University of Freiburg, schematically shown in Figure 2.

Bearing in mind the desired goal of performing high-target-number multiplexes, potentially containing several tens or more target-specific primer pairs and possibly probes, we have developed an assay based on the combination of ARMS primers and mediator probe chemistry. To date, the mediator probe chemistry has only been used in combination with standard primers, *i.e.* primers that are not mutation- or variant-selective and amplify a nucleic acid region irrespective of whether a mutation/SNP of interest is present therein. Consequently, to date, where a variant-specific detection would be of interest, the mediator probe would be designed such to partially overlap with the mutation or SNP of interest. However, we found that said approach required extensive optimization of the mediator probes in order to ensure their robust allele-selectivity. In particular, this would generally not allow reaching sensitivities of 1% or less for the detection of SNPs, making it unsuitable for sensitive mutation detection assays. We therefore modified the mediator probe chemistry-based assay in such a way that ARMS primers are used for the selective amplification of the mutant target molecules, and the mediator probes do not overlap with the mutation of interest. ARMS primers can contain e.g. wobbles and/or loops, which we hypothesized could allow in a complex multiplex setting for fine-tuning sensitivity/specificity outcomes for more challenging targets.

The results showing strong discriminative power of the presented herein approach are shown in Figure 3, illustrating the performance of a 3-plex assay that combines the use of ARMS primers and mediator probes for the highly sensitive detection of SNPs and indels in an EGFR gene. The results show that a titration series of synthetic mutant targets was robustly amplified and clearly detectable in a genomic wild type background of 10.000 copies. The 3-plex assay was able to detect down to 10 mutant target copies with sufficient discrimination from the Limit of Blank (LOB, indicated with a solid square in the figure) for some of the targets of the 3-plex assay.

### 3. Configuration of a prototype generic cartridge

The generic cartridge prototype was prepared based on a fluidic sample-processing cartridge proprietary to Biocartis NV and compatible with their automated molecular testing system Idylla^{™}. The standard cartridge is manufactured as a single disposable entity containing a sample entry port and multiple internal compartments for reagents and waste that communicate with a fluidic path for sample processing and nucleic acid isolation according to a strategy of choice. The fluidic path terminates in five independent nucleic acid PCR amplification chambers preloaded with amplification reagents and configured to accept a portion of the nucleic acids as isolated in upstream sections of the fluidic path. The amplification chambers are equipped with transparent walls that enable detection of signals generated during nucleic acid amplification such as PCR performed with light-emitting dyes signalizing the presence of a genetic target of interest. Once a sample is provided into the cartridge and the cartridge is fed into the Idylla^{™} system, the entire sample-to-result processing programme is orchestrated in a fully automated manner inside of the system, including biological sample disruption, nucleic acid isolation by means of e.g. either liquefaction or solid phase extraction, followed by target nucleic acid amplification, and signal detection.

For creating the first proof-of-principle generic cartridge prototypes, Idylla^{™} cartridges as described above were loaded with reagents and a buffer optimized for liquefaction of fixed solid tissue samples (e.g. FFPE samples), as described in EP2958997 in the name of Biocartis NV. Next, spot solutions of 5 differently-labeled unique generic reporters together with PCR reagents including dNTPs, Taq polymerase, etc., were spotted and dried in accordance with the manufacturer's protocol in each one of the 5 amplification chambers of the cartridge. The Mg²⁺ and buffering agents required for PCR were integrated in the buffer used for liquefaction.

Separately from the generic cartridge prototype, a mix of 61 target-specific oligonucleotide subsets was designed as follows. Each target-specific oligonucleotide subset of the 61 subsets in the mix contained one forward primer, one reverse primer, and one mediator probe. With the exception of the subset specific to KIF11 as the positive control target, forward primers were typically designed as allele-selective ARMS primers, *i.e.* 61 primers that bind specifically to one allele of interest. In this example, one specific allele is regarded as a target, even though it may concern the same gene as another allele targeted by another subset in the mix. Consequently, it is possible that different target-allele-specific subsets may share oligonucleotides having the same sequences, like in this example. Namely, the reverse primers and the mediator probes in each subset were mostly non-allele-selective, and rather designed to bind to a region in a gene close to the allele of interest. In this experiment, the mix of 61 target-specific oligonucleotide subsets contained 61 forward primers of different sequences (defining the 61 genetic targets comprising different genes and different alleles of the same gene), 9 different reverse primers, and 9 different mediator probes. This is because several reverse primers and mediator probes were designed to constitute part of subsets specific to a different allelic target in the same gene, the different alleles being discriminated by the specificity of the ARMS forward primers. The cartridge layout is given in columns 3-5 of Table 1 below, and the position in which 9 specific targets can be detected is indicated. Sequences of the oligonucleotides are shown in Table 2. The concentrations in the mix can be obtained from Biocartis NV upon request, but can be determined by the person skilled in the art.

**Table 1**

| **Target** | **Mediator Probe (in pool)** | **Generic Reporter (spotted in cartridge)** | **Amplification chamber** | **Detection Channel** |
|---|---|---|---|---|
| - | | 1 | A | 1 |
| - | | 2 | B | 1 |
| EGFR L858R | 3 | 3 | C | 1 |
| KRAS G12C | 4 | 4 | D | 1 |
| - | | 5 | E | 1 |
| EGFR G719A | 6 | 6 | A | 2 |
| EGFR S768I | 7 | 7 | B | 2 |
| BRAF V600E | 8 | 8 | C | 2 |
| - | | 9 | D | 2 |
| - | | 10 | E | 2 |
| - | | 11 | A | 3 |
| - | | 12 | B | 3 |
| HER2 ex20ins | 13 | 13 | C | 3 |
| - | | 14 | D | 3 |
| - | | 15 | E | 3 |
| EGFR ex19del | 16 | 16 | A | 4 |
| EGFR C797S | 17 | 17 | B | 4 |
| - | | 18 | C | 4 |
| - | | 19 | D | 4 |
| - | | 20 | E | 4 |
| KIF11 control | 21 | 21 | A, B, C, D, E | 5 |

**Table 2:**

| SEQ ID NO | oligo name | 5>3 sequence |
|---|---|---|
| 1 | 5EGFR_T21_7 | AAAAGATCAAAGTGCTGGC |
| 2 | 5EGFR_T20_7 | GAATTCAAAAAGATCAAAGTGCAGA |
| 3 | 5EGFR_T19_11 | AATTCAAAAAGATCAAAGTGCGGT |
| 4 | 5EGFR_T22_2 | CTCTTGAGGATCTTGAAGGATGA |
| 5 | 5EGFR_T23_13 | GCTCTCTTGAGGATCTTGTAGA |
| 6 | 5EGFR_T24_13 | CTCTTGAGGATCTTGATGGC |
| 7 | 5EGFR_T25_11 | CTCTTGAGGATCTTGAAGCATAC |
| 8 | 5EGFR_T26_13 | CTCTTGAGGATCTTGATGGG |
| 9 | 5EGFR_T27_13 | TCTCTTGAGGATCTTGATGGT |
| 10 | 5EGFR_T2_11 | ATTCCCGTCGCTATCAAAACA |
| 11 | 5EGFR_T3_11 | CCGTCGCTATCAAGACATCT |
| 12 | 5EGFR_T6_5 | CCGTCGCTATCAAGGAATCGAA |
| 13 | 5EGFR_T10_5 | CGTCGCTATCAAGGAATCTC |
| 14 | 5EGFR_T4_11 | TCGCTATCAAGGAACCAAC |
| 15 | 5EGFR_T17_10 | CGTCGCTATCAAGGTTCCG |
| 16 | 5EGFR_T11_6 | CCGTCGCTATCAAGGCATC |
| 17 | 5EGFR_T16_8 | GTCGCTATCAAGGAACCGAA |
| 18 | 5EGFR_T5_9 | CGTCGCTATCAAGGAACCATC |
| 19 | 5EGFR_T9_6 | CGTCGCTATCAAGGAAGCA |
| 20 | 5EGFR_T12_2 | CGTCGCTATCAAGGAGCCAAC |
| 21 | 5EGFR_T14_8 | CGTCGCTATCAAGGAATCATC |
| 22 | 5EGFR_T15_22 | TCCCGTCGCTATCAAAATATCT |
| 23 | 5EGFR_T8_6 | TCCCGTCGCTATCAAGTCT |
| 24 | 5EGFR_T13_10 | GTCGCTATCAAGGAACAGAA |
| 25 | 5EGFR_T45_5 | CCGTCGCTATCAAGGCTCC |
| 26 | 5EGFR_T46_7 | CGTCGCTATCAAGGATCCG |
| 27 | 5EGFR_T47_8 | GTCGCTATCAAGGAGCAAT |
| 28 | 5EGFR_S768I_3 | GAAGCCTACGTGATGGGCAT |
| 29 | 5FQEA_1 | CTCCAGGAAGCCTTCCAGGA |
| 30 | 5EGFR_C797S_4 | CTCATGCCCTTCGGCTC |
| 31 | 5EGFR_C797S_3 | GCTCATGCCCTTCGGCA |
| 32 | 5EGFR_T49_11 | GTCAAGATCACAGATTTTGGTCG |
| 33 | 5EGFR_T50_2 | GTCAAGATCACAGATTTTGGGCGT |
| 34 | 5EGFR_T51_3 | GATTTTGGGCTGGCCAAACA |
| 35 | 5BRAF_V600K/R/M | TAGGTGATTTTGGTCTAGCTTCAA |
| 36 | 5BRAF_V600E/D | GGTGATTTTGGTCTAGCTAGAGA |
| 37 | 5KRAS_G12C_1 | AACTTGTGGTAGTTGGAGCTT |
| 38 | 5KRAS_G12V_2 | AACTTGTGGTAGTTGGAGGTGT |
| 39 | 5KRAS_G12D_1 | AACTTGTGGTAGTTGGAGGTGA |
| 40 | 5HER2_T2_9 | GGAAGCATACGTGATGGCATAC |
| 41 | 5HER2_T3_2 | GGAAGCATACGTGATGGCTTAC |
| 42 | 5HER2_T6_3 | GGCTGGTGTGGGCTCCCCGG |
| 43 | **5EGFR_T68_8** | GAACCTCTAAGTCAAGAGCCATCTGT |
| 44 | 3EGFR_G719_1 | GCCTGTGCCAGGGACCT |
| 45 | 3EGFR_T1_1 | CCCCACACAGCAAAGCAGAA |
| 46 | 3EGFR_S768I_1 | GTGGAGGTGAGGCAGATGC |
| 47 | 3EGFR_C797S_1 | CACACACCAGTTGAGCAGGTACT |
| 48 | 3EGFR_T48_6 | CTTACTTTGCCTCCTTCTGC |
| 49 | 3BRAF_V600_1 | CACAAAATGGATCCAGACAACTG |
| 50 | 3KRAS_G12C/V/D_1 | CATATTCGTCCACAAAATGATTCTG |
| 51 | 3HER2_T1_7 | GCTGCACCGTGGATGTCA |
| 52 | **3EGFR_T68_8** | GACATACCTGGAAAAATGGAACC |
| 53 | 5EGFR_G719_MP3_MHS1_M1 | CCGATCTACGTCGAGCGCGTTCGGCACGGTGTATAAGGTAAGGT/3Phos/ |
| 54 | 5EGFR_Ex19del_MP1_MHS2_M1 | GCGAGTCTTACGCTCGGACAAGGAAATCCTCGATGTGAGTTTCTG/3Phos/ |
| 55 | 5EGFR_S768I_MP1_MHS5_M2 | GTAGATGCATAGCCTACCGGACAACCCCCACGTGTGCCG/3Phos/ |
| 56 | 5EGFR_C797S_MP4_MHS8_M4 | TGGGGAGTTACTGTACCGATGGACTATGTCCGGGAACACAAAGACA/3Phos/ |
| 57 | 5EGFR_L858R_MP1_MHS9_M4 | GCAACATCAGATATCGCGTGCGGAAGAGAAAGAATACCATGCAGAA/3Phos/ |
| 58 | 5BRAF_V600_MP2_MHS12_M2 | CGAGCTAACTACGGTACGTCTCGATGGAGTGGGTCCCATCAGTTT/3Phos/ |
| 59 | 5KRASG12_MP1_MHS19_M1 | GTGTCACTCGATTACGCGCAAGAGTGCCTTGACGATACAGCTA/3Phos/ |
| 60 | 5HER2_MP1_MHS14_M3 | CGGAGGACTGTCGCGTATATGCCCAGAAGGCGGGAGACATA/3Phos/ |
| 61 | **5KIF11_MP1_MHS15_M2** | GTACTGAGTCGCCGAATCTGGTGTGGATTGTTCATCAATTGGCG/3Phos/ |

Each mediator sequence (or "free mediator" comprising or consisting of a unique generic sequence tag) complementary to exactly one of the generic reporter probes (*i.e.* complementary to the unique generic sequence tag binding site of the generic reporter molecule) as spotted in the amplification chambers of the generic cartridge prototype, was covalently coupled to a target specific sequence to together form one of the 9 mediator probes. The mediator sequences can e.g. be designed as nullomers (*i.e.* a scrambled sequence that does not occur in the human genome) of 10-30 nucleotides in length, but also non-nullomers can be considered. The lengths of the mediator sequences can vary but are considered to be in an acceptable range if they are predicted to give a specific signal by interacting with their corresponding generic reporter and to not cross-react with other generic reporters. Examples of such mediator sequences can be found in Wadle et al. (2015, Biomolecular Detection and Quantification 7:1-8, Real-time PCR probe optimization using design of experiments approach). Sequences as used herein as mediator sequences can also be obtained from Biocartis upon request.

For verifying if the 61-plex with mediator chemistry is suitable to generate target-specific signals in the generic cartridge prototype, the following components were added together into the lysis chamber of each prototype cartridge via its sample entry port:
(i) 27 ul of the mix of the 61 target-specific oligonucleotide subsets comprising primer pairs and mediator probes;
(ii) a mock sample material selected from an FFPE slice, genomic DNA extract, or milliQ water corresponding to no-sample control;
(iii) synthetic target containing a mutation of interest (10-50-100 copies per PCR chamber) or no synthetic target (negative control).

The generic prototype cartridges were then inserted into the Idylla^{™} instruments and the fully automated tests were initiated. During these tests, the Idylla^{™} platform executes pumping of the sample preparation buffer into the lysis chamber and applies heating and high frequency ultrasound (HiFU) treatment to the contents of the lysis chamber in order to obtain a homogenous liquefact. In a next step, the liquefact is heat-inactivated by slow pumping through a heated area followed by transfer of a portion of the heat-inactivated liquefact comprising nucleic acid targets and the mix of target-specific oligonucleotide subsets to each one of the 5 parallel amplification chambers. At this point, the PCR cycling protocol adapted for the 61-plex with the oligonucleotide mix is started, which in the presented herein setting results in generation of free mediators if appropriate target genes or alleles are present in the given liquefact portion. The free mediators hybridize to their respective complementary general reporter probes as spotted in the amplification chambers. The hybridization events result in the generation of fluorescence signals that are measured during the annealing/elongation steps, followed by standard post-processing to correct the data for offset and drift, and for the determination of the first cycle at which amplification can be detected. A Decision Tree with 2 simple parameters was used to remove curves that either had a fluorescence signal that was too low (cf. ratio of signal at the plateau versus at the baseline should be >0.1), or had a Cq that was not in the expected range (cf. 15<Cq<36).

To test the sensitivity, the above procedure was performed using a synthetic target HER2 ex20ins at titration series of 0-10-50-100 copies per PCR chamber (corresponding to 0.0-0.1-0.7-1.4% mutant copies when used in a sample containing 7000 genomic copies per PCR chamber). The synthetic targets were admixed with either a high-background clinical sample containing 7000 genomic copies per PCR chamber or 1000 copies of genomic DNA per PCR chamber, both confirmed upfront to be negative for the mutations of interest. Exemplary obtained amplification curves are shown in Figures 4 and 5

The sequences of the amplification primers, mediator probes, and generic reporters used in the above-described example are provided in Table 3.

**Table 3**

| SEQ ID NO | oligo name | 5>3 sequence |
|---|---|---|
| 40 | 5HER2_T2_9 | GGAAGCATACGTGATGGCATAC |
| 51 | 3HER2_T1_7 | GCTGCACCGTGGATGTCA |
| 60 | 5HER2_MP1_MHS14_M3 | CGGAGGACTGTCGCGTATATGCCCAGAAGGCGGGAGACATA/3Phos/ |

Firstly, the data unexpectedly show that with the presented herein methodology, it is possible to detect down to 10 copies/PCR of a marker, as exemplified by HER2 ex20ins in Figure 4. The method is consequently surprisingly sensitive, given the multiplexing complexity. Furthermore, as no false-positives were detected across over 50 prototype cartridges tested, it can be concluded that the present method meets stringent specificity criteria as well.

Further, the data show that it is possible to detect one specific marker in a specific chamber, even though the marker was amplified across all of the amplification chambers in parallel, identical multiplex reactions with primer pairs and mediator probes provided together with the biological sample. For example, EGFR G719A is detected in channel 2 from chamber A, but not in the other chambers; EGFR S768I is detected in channel 2 from chamber B, but not in the other chambers. Similarly, EGFR L858R is detected in channel 1 from chamber C, but not in the other chambers; KRAS G12C is detected in channel 1 from chamber D, but not in the other chambers. The data further show that it is possible to discriminate between two mutants within the same chamber. Namely, C797S is detected in channel 4 from chamber B, and not in the other channels from chamber B; EGFR S768I is detected in channel 2 from chamber B, and not in the other channels from chamber B. Lastly, the data also importantly show that it is possible to detect multiple markers at the same time in a single amplification chamber using different generic reporters. To illustrate this, each discussed herein target was always being detected in combination with the positive control genomic reference gene, which was used as a sample processing control in all amplification chambers.

### 4. Development of an improved primer-probe system for multiplex amplification

In some instances, it may be valuable to discriminate neighboring mutations, as they may lead to a different clinical action. An example of this includes the need to discriminate KRAS G12C from other KRAS G12 or G13 mutations, if it is desired to use the mutation assay for the detection of KRAS mutant tumors that could respond to a specific KRAS G12C-targeting therapy. Traditionally, in a sample-to-result platform with multiple chambers, this is arranged by spotting the ARMS primer for KRAS G12C amplification in a different chamber than the ARMS primers for the other KRAS G12 or G13 mutations. However, when working in a situation where all target-specific oligonucleotides are added together through the sample entry port, it is not possible to keep the spatial separation between the particular ARMS primers. We therefore further modified the ARMS primers to include a stem-loop structure, in which the stem can optionally be composed of the target sequence. We further modified the mediator probe in such a way that it at least partially overlaps with the modified ARMS primer, while in the standard mediator probe design the probes are always downstream of the primer used for amplification. The concept is shown in Figure 6 for a situation where the binding site of the mediator probe falls completely within the ARMS primer, but it can be envisioned that part of the binding site of the mediator probe falls outside of the ARMS primer. The 3' component of the stem of the ARMS primer can have a sequence that is derived from the target sequence, or can be another sequence. When the target is not amplified, the mediator probe cannot bind to the target sequence and hence cannot create a signal. The mediator probe also cannot bind to its complementary sequence within the ARMS primer, which is inside of the stem-loop configuration and hence inaccessible for the mediator probe. When the target is amplified, the stem structure can be unfolded by the polymerase, thereby creating a binding site for the mediator probe. Once bound, the free mediator is released as the other primer is extended, and the free mediator can bind to the spotted generic reporter and create a signal.

It will be appreciated by the person skilled in the art that the use of 2 or more mediator probes increases the detection potential considerably. For instance, in case of 2 mediator probes, 4 different conditions can be differentiated from each other, e.g.

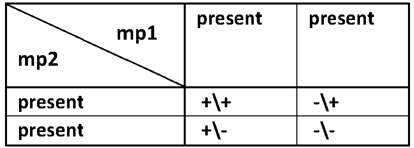

The results are shown in Figure 7, indicating the robustness of the concept as well as the ubiquitously usefulness, i.e. the concept is not confined to the generic cartridges of the present invention but can be broadly used in multiplex amplification reactions.

### 5. Development of FuseTag

In a further development of the primer-probe system described in Example 4, the inventors set out to simplify the system in order to further increase robustness, and at the same time reduce costs. This was achieved by combining the stem-loop primer and mediator probe 2 into one "FuseTag" primer thus reducing the number of components compared to the improved primer-probe system of Example 4. Moreover, the FuseTag primers do not contain modifications, such as the -costly- polymerase extension blocker (indicated by the square box in the figures), which enables fast synthesis and iterations. The FuseTags allow discrimination of neighboring markers when working with a FLEX Generic Detection cartridge.

Figure 6C shows the "FuseTag" concept enabling the discrimination of neighboring markers. The modified forward ARMS primer ("FuseTag" primer) now includes from 5' to 3': a first portion, wherein the first portion comprises a unique generic sequence tag ("UGST"), which when released is indicated as free mediator 2 in Figure 6C; a stem-loop structure; and a second portion, wherein the second portion is complementary to the target (and preferably allele-specific). When the specific target is not amplified by the FuseTag primer, the unique generic sequence tag (mediator 2) is not released and hence cannot create a signal. However, the mediator probe 1 can still be hydrolyzed by another forward primer present in the multiplex reaction mixture.

Extension of the FuseTag primer by a polymerase leads to hydrolysis of the target specific component of the mediator probe 1 and liberation of free mediator 1. In the subsequent PCR cycle extension of the reverse (RE) primer by a polymerase leads to hydrolysis of the double-stranded part of the FuseTag primer resulting in the liberation of the unique generic sequence tag (free mediator 2).

Both the free mediator 1 and the free mediator 2 can bind to their corresponding universal reporter. Note that the fluorophore and quencher can be swapped (not shown in Figure 6C). Upon extension of the free mediator, a fluorescent signal is created by displacement of the quencher or fluorophore modification and/or hydrolysis of the quencher or fluorophore-linked nucleotides (not shown). Note that the non-hydrolysed mediator probe and generic reporter cannot be extended by the polymerase (as symbolically indicated by a square).

The concept was first tested in a singleplex set-up. Figure 10 shows the performance for mutation detection using FuseTag primers in singleplex PCR, *i.e.* containing only the primers that are needed to amplify 1 target, in a 96-well format qPCR instrument for four different targets. The targets were added as synthetic mutant targets at 200 copies in the PCR reaction, which always contained 2,000 copies of genomic wild-type DNA, as well as the universal reporters, and the polymerase, dNTPs and PCR salts (incl. MgCl₂). Four different combinations with different targets were tested, *i.e.* BRAF V600E, EGFR E709K, EGFR S768I and EGFR L861Q. The sequences of the various FuseTag primers are shown in Table 4.

**Table 4**

| SEQ | name | FuseTag |
|---|---|---|
| | Top left: BRAF V600E | |
| 62 | FEB_FMG_BRAF_T2_F045 | actagtcatcgcgtaatcGCcggggccccTTTTTTggggc cccgGCCAGTAAAAATAGGTGATTTTGGTCTAGCTAGAGA |
| 49 | 3BRAF_V600_1 (REV) | CACAAAATGGATCCAGACAACTG |
| | Top right: EGFR E709K | |
| 63 | FEB_FMG_EGFR_T23_F053 | cgtgtctgaagcgcgCGAggggccccTTTTTTggggcccc TCGACCAAGCTCTCTTGAGGATCTTGATGA |
| 44 | 3EGFR_G719_1 (REV) | GCCTGTGCCAGGGACCT |
| | Bottom left: EGFR S768I | |
| 64 | FEB_FMG_EGFR _T30_F043 | gctatctgtacgcgattcGACggggccccTTTTggggccc cGTCCAGGAAGCCTACGTGATGGCGAT |
| 46 | 3EGFR_S768I_1 (REV) | GTGGAGGTGAGGCAGATGC |
| | Bottom right: EGFR L861Q | |
| 65 | FEB_FMG_EGFR_T51_F042 | tcgttctgggctctacGACggggccccTTTTggggccccG TCCAGATTTTGGGCTGGCCAATCA |
| 48 | 3EGFR_T48_6 (REV) | CTTACTTTGCCTCCTTCTGC |
| | | |
| 66 | FEB_FMG_BRAF_T2_F041 | actagtcatcgcgtaatcGCcggggccccTTTTTTggggc cccgGCATAGGTGATTTTGGTCTAGCTACAGA |
| | KRAS G12C/D | |
| 67 | 5KRAS_G12C_E4_MP1_MHS19 | GTGTCACTCGATTACGCGCTCCAACTACCGACGTATCGG |
| 68 | 5KRAS_G12C_INS1_E4_S3_G25 | GCTGAAAATGACTGAATATAAACTTACCCCGATACGTCGG TAGTTGGAGCTT |
| 50 | 3KRAS_G12C/V/D_1 (REV) | CATATTCGTCCACAAAATGATTCTG |

(no probes were used in these FuseTag experiments)

The amplification results of 200 copies in a gDNA background are indicated by black lines in Figure 10. Control reactions are indicated by grey lines where genomic wild-type DNA was present, but no synthetic mutant targets were added. The results demonstrate the target specificity over the negative control.

The experiment was repeated with KRAS G12C, two mutant variants of EGFR C797S, two mutant variants of EGFR T790M, and EGFR G719S, which all give essentially the same results, *i.e.* target specificity over the negative control (data not shown).

In conclusion, the results evidence the general feasibility of the "FuseTag" concept as a generic tag that can be used target independently.

To further demonstrate the ubiquitous practicality of the "FuseTag" concept, the performance for mutation detection in a multiplex PCR was examined. In particular, a mixture comprising an oligonucleotide pool containing primers that are needed to amplify multiple targets, as well as mediator probes in a 96-well format qPCR instrument was tested. The targets were added as synthetic mutant targets at various concentrations together with the mixture containing primers and mediator probes, and 10³ copies of genomic wild-type DNA. The generic reporters, polymerase, dNTPs and PCR salts (including MgCl₂) were added to each reaction together with a liquefaction buffer containing components that would be required to release DNA from a FFPE sample. The "FuseTag" primer FEB_FMG_BRAF_T2_F041 is shown in Table 4 (other components are not shown)

The amplification results of 500 (triangles), 100 (filled circles), and 20 (diamonds) copies in a gDNA background are indicated in Figure 11. Control reactions are indicated by filled squares, where genomic wild-type DNA was present, but no synthetic mutant targets were added (0 copies). The results demonstrate the target specificity over the negative control in a multiplex context.

In conclusion, the results demonstrate the efficacy and specificity of the "FuseTag" concept in broad range of applications.

### 6. Development of quality control (QC) amplification using KIF11 for DNA fragmentation assessment

For certain specific applications, like determination of nucleic acid fragmentation or assessment of contamination of short cell-free DNA from plasma with genomic DNA from white blood cells, we have initially envisaged a quality control triplex based on three different housekeeping genes including ABCB, RNaseP, and TFRC. However, having observed that these and other tested genes did not exhibit satisfactory level of stability, and after having identified KIF11 as a surprisingly mutation-free and stable genomic region, we have redeveloped the initial triplex fragmentation assay based on different exons of KIF11. The switch to a single stable region was hypothesized as beneficial to avoid copy number variations, which could occur if several different reference genes were used. Consequently, different exons of KIF11 were used to develop primers and probes for amplification and detection, respectively, of three amplicons of discernably different lengths being 62 bp, 98 bp, and 136 bp. The conditions of cycling can be easily determined by the person skilled in the art, nonetheless the conditions of cycling inside of Idylla^{™} cartridges can be obtained from Biocartis upon request. The QC triplex was tested on samples with different degree of DNA fragmentation, including DNA derived from FFPE samples, nucleosomal DNA from blood, intact genomic DNA, etc. As shown in Figure 8, the KIF11-based QC triplex gives a reliable indication of the degree of fragmentation, and hence the quality of the DNA present in a particular sample.

The sequences of the amplification primers, mediator probes, and generic reporters used in the above-described example are provided in Table 5.

**Table 5**

| ***62bp- KIF11*** | non-coding sequence after KIF11 gene | SEQ:* |
|---|---|---|
| FW primer: | CGGGAAGTCAGACGGGTCA | 69 |
| Rev primer: | GAGAGGTACCGGCAGGAGCA | 70 |
| Probe: | CCCCAGACCCCGGCTGCAGCGC | 81 |
| ***98bp- KIF11*** | intron 6 | |
| FW primer: | GGAAGCCCAGGATAAAATGTGGCT | 71 |
| Rev primer: | AAGTGCCTCAGGGACCCCTTCA | 72 |
| Probe: | CTAAGGAGGTAAGCCTCAGAGCGTCCCATTCC | 82 |
| ***136bp- KIF11*** | intron 6 | |
| FW primer: | GCCATAGTCTCTTCCCTAGCCCCAT | 73 |
| Rev primer: | GCCAATTACCAATGACCCCTCCTT | 74 |
| Probe: | CGTGACCCACATACCCTGACCCACCCC | 83 |
| ***89bp- KIF11*** | Exon 21- Intron 21 boundary | |
| FW primer: | GAACCTCTAAGTCAAGAGCCATCTGT | 75 |
| Rev primer: | GACATACCTGGAAAAATGGAACC | 76 |
| Mediator Probe: | ACTGAGTCGCCGAATCGCTGGTGTGGATTGTTCATCAATTGGCGG/3Pho s/ | 84 |
| Universal reporter: | /5Quasar670/CAGCCGGCCAAGACGCGCCGGCT[T(BHQ2)]GGCGAT TCGGCGACTCAGTACTATCAG /3SpC3/ | 87 |
| ***204bp- KIF11*** | Exon 8 | |
| FW primer: | GCCGTTCTGGAGCTGTTGATA | 77 |
| Rev primer: | GAGAGATGCAGGAGAAATTGTTGC | 78 |
| Mediator Probe: | CTCGTCTGCTTGTACACTGA CTCGGGAAGCTGGAAATATAAATCAATC/3Phos/ | 85 |
| Universal reporter: | /5Quasar705/CAGCCGGCCAAGACGCGCCGGCTT[T(BHQ- 2)]GGGATCAGTGTACAAGCAGACGAGAA /3SpC3/ | 88 |
| ***82bp- KIF11*** | Exon 21- Intron 21 boundary | |
| Fw primer: | TCTAAGTCAAGAGCCATCTGTA | 79 |
| Rev primer: | GATATGACATACCTGGAAAAATGGAA | 80 |
| Probe: | /5ATTO532N/ACCCCGCCAATTGATGAACAATCCA/3BHQ-1/ | 86 |

| | | |
|---|---|---|
| SEQ:* - SEQ ID NO: | | |

### 7. Determination of DNA fragmentation using KIF11 in mediator chemistry-based qPCR

The next step was the development of DNA fragmentation control qPCR amplification based on KIF11 and its detection with the mediator chemistry readout adapted for the prototype generic cartridge as described above. To do so, a short (82 bp) and a long (204 bp) PCR amplicon targeting different exons of the KIF11 were designed together with two corresponding to them mediator probes. Each of the mediator probes was designed to target its specific generic reporter conjugated with different light-emitting dyes based in channel 5 and channel 1, respectively. Both of the reporters were provided in the same amplification chamber of each prototype cartridge, such that qPCR signals associated with the two amplicons and mediators could be detected in the same reaction. Determination of the Cq difference (delta Cq) between the long and short amplicon qPCR curves was used as a measure of DNA fragmentation. Performance of the "KIF11 DNA fragmentation duplex" was determined on a set of clinical FFPE samples, of which the DNA fragmentation level was previously determined using an orthogonal ddPCR-based method. As a positive control, the assay was also tested on high quality genomic DNA (gDNA) derived from white blood cells (Promega), which is expected to be unfragmented. The results are shown in Figure 9, wherein the lines with circles represent the short amplicon and the regular lines represent the long amplicon. Cq values were determined using a threshold (horizontal line). The results clearly show that the delta Cq value between the long and short KIF11 amplicons as detected by the mediator chemistry, strongly correlates with the degree of DNA fragmentation in the evaluated FFPE samples and that the developed herein fragmentation duplex is readily usable for generic cartridges of the presented herein concept. Such duplex is very well suited to not only provide a positive amplification control for personalized oligonucleotide panels, but, thanks to its ability to detect fragmented samples, it also can provide an indication of the reliability of the final results in function of the sample quality. Then, as extensive fragmentation in FFPE samples correlates with deamination artefacts, due to both being caused by formalin fixation, the present KIF11 duplex assay may also be suitable for prediction of deamination artefacts. Lastly, for cfDNA-based assays from plasma, like the ones targeting circulating foetal, tumor, or organ donor DNA-target panels, the presented here fragmentation assays are likely also suitable to provide information on e.g. contamination with genomic DNA from white blood cells in a plasma sample.

In conclusion, we provided herein a working proof-of-principle demonstration of a highly unusual approach to a fast-tract and extremely versatile diagnostic assay and product development. We believe that the presented herein concepts have an enormous potential to open an entire new venue for rapid, customized, and individualized molecular testing strategies as it eliminates the need for the design, manufacturing and QC release of customized cartridges. In particular, the disclosed herein methods, kits, kits of parts, and uses, can substantially reduce the development costs and the time for bringing new assays to patients. Especially now, such development acceleration is extremely called for, as shown by a sudden outbreak of a global pandemic in 2020, which stalled and delayed production of essential medical and diagnostic products worldwide, delaying treatment decisions and access to adequate medical care for many in need.

## Claims

1. A method for quantifying the number of target nucleic acids in a sample in relation to KIF11 nucleic acids in said sample, comprising:
1. amplifying KIF11 nucleic acids comprised in the sample using a KIF11-specific primer pair in a KIF11 amplification reaction,
i. wherein each of the members of the KIF11-specific primer pair is -independently from each other- complementary to a KIF11 region located in an exon, intron, or the non-coding sequence of the KIF11 gene;
ii. wherein the KIF11 amplification reaction is performed in the presence of a KIF11 detectable probe;
2. detecting a signal produced by the KIF11 detectable probe in the KIF11 amplification reaction;
3. quantifying the signal of 2.;
4. amplifying the target nucleic acids from the sample using a target-specific primer pair in a target amplification reaction;
wherein the target amplification reaction is performed in the presence of a target detectable probe; and
5. detecting a signal produced by the target detectable probe in the target amplification reaction;
6. quantifying the signal of 5.;
7. normalizing the quantified signal of 6. to the quantified signal of 3., thereby quantifying the number of target nucleic acids in a sample in relation to KIF11 nucleic acids in the sample.

2. The method according to claim 1, wherein said KIF 11 region is located in any of exons 6, 8, 18, 21, intron 6, exon 21 - intron 21 boundary or a non-coding region of the KIF11 gene.

3. The method according to claim 1 or 2, wherein the KIF-specific primer pair is a primer pair selected from the following:
i. SEQ ID NO:s 69 and 70;
ii. SEQ ID NO:s 71 and 72;
iii. SEQ ID NO:s 73 and 74;
iv. SEQ ID NO:s 75 and 76;
v. SEQ ID NO:s 77 and 78; and
vi. SEQ ID NO:s 79 and 80.

4. The method according to any of claims 1-3, wherein the KIF11 detectable probe is selected from SEQ ID NO:s 81 to 88.

5. The method according to any one of claims 1-4, wherein the probes are fluorescently labelled.

6. The method according to any one of claims 1-5, wherein the target nucleic acid and the KIF11 gene are amplified in the same amplification reaction and/or obtained from the same sample.

7. The method according to any one of claims 1-6, wherein the amplification reaction is a polymerase chain reaction (PCR), preferably a Quantitative PCR (qPCR).

8. The method according to claim 7, wherein the result of the qPCR is reported as ΔCq value or double ΔCq value.

9. The method according to any one of the claims 1-8, wherein the sample is a fresh tissue sample or a frozen or FFPE sample.

10. Use to amplify a region of the genomic reference gene KIF11 of a kit comprising primers and instructions comprising an amplification protocol and analysis of the results, wherein the primers are a primer pair selected from the following:
i. SEQ ID NO:s 69 and 70;
ii. SEQ ID NO:s 71 and 72;
iii. SEQ ID NO:s 73 and 74;
iv. SEQ ID NO:s 75 and 76;
v. SEQ ID NO:s 77 and 78; and
vi. SEQ ID NO:s 79 and 80,
preferably the amplified region is detected by probes selected from SEQ ID NO:s 81-86, preferably via generic reporter selected from SEQ ID NO: 87 and 88.

11. A method for determining gDNA contamination in a sample, comprising:
1. amplifying KIF11 nucleic acid comprised in the sample using a first and a second KIF11-specific primer pair in a KIF11 amplification reaction,
a. wherein at least one member of the first KIF11-specific primer pair is complementary to a KIF11 intron or the non-coding sequence of the KIF11 gene;
b. wherein the amplification reaction using the first KIF11 specific primer pair is performed in the presence of a first KIF11 detectable probe;
c. wherein each of the members of the second KIF11-specific primer pair is located in a KIF11 exon;
d. wherein the amplification reaction using the second KIF11-specific primer pair is performed in the presence of a second KIF11 detectable probe;
2. detecting signals produced by the first and second KIF11 detectable probes in the first and second KIF11 amplification reactions;
3. quantifying the signals of the first and second KIF11 amplification reactions;
4. normalizing the quantified signal of the first amplification reaction to the quantified signal of the second amplification signal, thereby determining gDNA contamination in the sample, preferably the sample is a mitochondrial DNA, cDNA, mRNA, rRNA, tRNA, hnRNA, microRNA, IncRNA, cfDNA, cell-free tumor DNA or an siRNA sample.

12. A method for determining integrity of nucleic acids in a sample, comprising:
1. amplifying KIF11 nucleic acid comprised in the sample using a first and a second KIF11-specific primer pair in a KIF11 amplification reaction,
a. wherein each of the members of the first KIF11-specific primer pair is -independently from each other- complementary to a KIF11 region located in an exon, intron, or the non-coding sequence of the KIF11 gene;
b. wherein the amplification reaction using the first KIF11 specific primer pair is performed in the presence of a first KIF11 detectable probe;
c. wherein each of the members of the second KIF11-specific primer pair is - independently from each other- complementary to a KIF11 region located in an exon, intron, or the non-coding sequence of the KIF11 gene;
d. wherein the amplification reaction using the second KIF11-specific primer pair is performed in the presence of a second KIF11 detectable probe;
2. determining the threshold values in each of said nucleic acid amplification reactions by
i. measuring, at a plurality of different times during the amplification reaction, at least one signal whose intensity is related to the quantity of a nucleic acid sequence being amplified in the reaction; and
ii. determining the cycle number associated with the characteristic of the derivative, which represents the threshold value;
3. comparing the threshold values (threshold cycle numbers) of the first and second KIF11 amplification reactions;
4. wherein the difference between the threshold cycle numbers (ΔCq) of the first and the second KIF11 amplification reactions is a measure for the integrity of genomic DNA.

13. The method according to claim 12, wherein the amplicons generated by the amplification reaction of the first and second KIF11-specific primer pairs are sufficiently far located to not interfere.

14. The method according to claim 12 or 13, wherein the amplicons are at least 600 basepairs (bp), such as at least 700 bp, 800 bp, 900 bp or even more, such as at least 1 kilobasepairs.

15. A method for determining gDNA fragmentation, comprising
i. generating 3 (first, second and third) KIF11 amplicons of discernably length by first, second and third amplification reactions, preferably by PCR;
ii. determining the Cq values of said first, second and third amplification reactions;
iii. comparing the Cq of said first, second and third amplification reactions;
wherein the differences in Cq values between said first, second and third amplification reactions is an indication of the gDNA fragmentation; or
i. generating a first KIF11 amplicon ("short") by a first amplification reaction; and
ii. generating a second KIF11 amplicon ("long") by a second amplification reaction;
iii. determining the Cq values of said first and said second amplification reaction;
iv. determining the ΔCq value of said first and said second amplification reaction;
wherein the ΔCq is an indication of gDNA fragmentation.

16. A method for quality controlling the processing, isolation and amplification process comprising:
i. sample processing;
ii. nucleic acid isolation;
iii. generating 3 KIF11 amplicons of discernably different lengths by amplification reactions;
iv. determining the Cq values of each of said KIF11 amplicons;
wherein the Cq values of the KIF11 amplicons are a measure for the quality control of the processing, isolation and amplification.

17. A method according to any of the claims 1-9, comprising at least one primer from the target-specific primer pairs selected from the following:
(1) an EGFR primer pair, wherein the
EGFR forward primer is selected from SEQ ID NO:s 1-34, and
EGFR reverse primer is selected from SEQ ID NO:s 44-48;
preferably the EGFR amplicon is detected by any one of SEQ ID NO:s 53-57;
(2) a BRAF primer pair, wherein the
BRAF forward primer is selected from SEQ ID NO:s 35 and 36, and
BRAF reverse primer is SEQ ID NO: 49;
preferably the BRAF amplicon is detected by SEQ ID NO: 58;
(3) a KRAS primer pair, wherein the
KRAS forward primer is selected from SEQ ID NO: 37-39 and 67-68, and
KRAS reverse primer is SEQ ID NO: 50;
preferably the KRAS amplicon is detected by SEQ ID NO: 59;
(4) a HER2 primer pair, wherein the
HER2 forward primer is selected from SEQ ID NO: 40-42, and
HER2 reverse primer is SEQ ID NO: 51;
preferably the HER2 amplicon is detected by SEQ ID NO: 60.

## Patentansprüche

1. Verfahren zur Quantifizierung der Anzahl an Ziel-Nukleinsäuren in einer Probe im Verhältnis zu KIF11-Nukleinsäuren in dieser Probe, umfassend:
1. Amplifizierung von KIF11-Nukleinsäuren, die in der Probe umfasst sind, unter Verwendung eines KIF11-spezifischen Primerpaares in einer KIF11-Amplifikationsreaktion,
i. wobei jeder der Bestandteile des KIF11-spezifischen Primerpaares - unabhängig voneinander - komplementär zu einer KIF11-Region ist, die in einem Exon, Intron oder der nicht-kodierenden Sequenz des KIF11-Gens liegt;
ii. wobei die KIF11-Amplifikationsreaktion in Anwesenheit einer KIF11-nachweisbaren Sonde durchgeführt wird;
2. Detektion eines Signals, dasdurch die KIF11-nachweisbare Sonde in der KIF11-Amplifikationsreaktion erzeugt wird;
3. Quantifizierung des Signals von 2.;
4. Amplifizierung der Ziel-Nukleinsäuren aus der Probe unter Verwendung eines Ziel-spezifischen Primerpaars in einer Ziel-Amplifikationsreaktion;
wobei die Ziel-Amplifikationsreaktion in Anwesenheit einer Ziel-nachweisbaren Sonde durchgeführt wird; und
5. Detektion eines Signals, das durch die Ziel-nachweisbare Sonde in der Ziel-Amplifikationsreaktion erzeugt wird;
6. Quantifizierung des Signals von 5.;
7. Normalisierung des quantifizierten Signals von 6. auf das quantifizierte Signal von 3., dadurch Quantifizierung der Anzahl an Ziel-Nukleinsäuren in einer Probe im Verhältnis zu KIF11-Nukleinsäuren.

2. Verfahren nach Anspruch 1, wobei die KIF11-Region in einem der Exons 6, 8, 18, 21, Intron 6, Exon 21-Intron 21-Grenze oder einer nicht-kodierenden Region des KIF11-Gens lokalisiert ist.

3. Verfahren nach Anspruch 1 oder 2, wobei das KIF-spezifische Primerpaar ein Primerpaar ausgewählt aus den folgenden ist:
i. SEQ ID NO:s 69 und 70;
ii. SEQ ID NO:s 71 und 72;
iii. SEQ ID NO:s 73 und 74;
iv. SEQ ID NO:s 75 und 76;
v. SEQ ID NO:s 77 und 78; und
vi. SEQ ID NO:s 79 und 80.

4. Verfahren nach einem der Ansprüche 1-3, wobei die KIF11-nachweisbare Sonde ausgewählt ist aus SEQ ID NO:s 81 bis 88.

5. Verfahren nach einem der Ansprüche 1-4, wobei die Sonden fluoreszenzmarkiert sind.

6. Verfahren nach einem der Ansprüche 1-5, wobei die Ziel-Nukleinsäure und das KIF11-Gen in derselben Amplifikationsreaktion amplifiziert und/oder aus derselben Probe erhalten werden.

7. Verfahren nach einem der Ansprüche 1-6, wobei die Amplifikationsreaktion eine Polymerase-Kettenreaktion (PCR) ist, bevorzugt eine quantitative PCR (qPCR).

8. Verfahren nach Anspruch 7, wobei das Ergebnis der qPCR als ΔCq-Wert oder doppelter ΔCq-Wert angegeben wird.

9. Verfahren nach einem der Ansprüche 1-8, wobei die Probe eine frische Gewebeprobe oder eine gefrorene oder FFPE-Probe ist.

10. Verwendung eines Sets zur Amplifizierung einer Region des genomischen Referenzgens KIF11, umfassend Primer und Anweisungen, umfassend ein Amplifikationsprotokoll und Analyse der Ergebnisse, wobei die Primer ein Primerpaar ausgewählt aus den folgenden sind:
i. SEQ ID NO:s 69 und 70;
ii. SEQ ID NO:s 71 und 72;
iii. SEQ ID NO:s 73 und 74;
iv. SEQ ID NO:s 75 und 76;
v. SEQ ID NO:s 77 und 78; und
vi. SEQ ID NO:s 79 und 80,
bevorzugt wird die amplifizierte Region durch Sonden ausgewählt aus SEQ ID NO:s 81-86 detektiert,
bevorzugt über generische Reporter ausgewählt aus SEQ ID NO:s 87 und 88.

11. Verfahren zur gDNA-Kontaminations-Bestimmung in einer Probe, umfassend:
1. Amplifizierung von KIF11-Nukleinsäuren, die in der Probe umfasst sind, unter Verwendung eines KIF11-spezifischen Primerpaares in einer KIF11-Amplifikationsreaktion,
a. wobei mindestens ein Bestandteil des ersten KIF11-spezifischen Primerpaares komplementär zu einem KIF11-Intron oder der nicht-kodierenden Sequenz des KIF11-Gens ist;
b. wobei die Amplifikationsreaktion, die das erste KIF11-spezifische Primerpaar verwendet, in Anwesenheit einer ersten KIF11-nachweisbaren Sonde durchgeführt wird;
c. wobei jeder der Bestandteile des zweiten KIF11-spezifischen Primerpaars in einem KIF11-Exon lokalisiert ist;
d. wobei die Amplifikationsreaktion, die das zweite KIF11-spezifische Primerpaar verwendet, in Anwesenheit einer zweiten KIF11-nachweisbaren Sonde durchgeführt wird;
2. Detektion von Signalen, die durch die ersten und zweiten KIF11-nachweisbaren Sonden in den ersten und zweiten KIF11-Amplifikationsreaktionen erzeugt werden;
3. Quantifizierung der Signale der ersten und zweiten Amplifikationsreaktionen;
4. Normalisierung des quantifizierten Signals der ersten Amplifikationsreaktion auf das quantifizierte Signal der zweiten Amplifikationsreaktion, dadurch Bestimmung der gDNA-Kontaminierung in der Probe, bevorzugt ist die Probe eine mitochondriale DNA, cDNA, mRNA, rRNA, tRNA, hnRNA, microRNA, IncRNA, cfDNA, zellfreie Tumor-DNA oder eine siRNA-Probe.

12. Verfahren zur Integritäts-Bestimmung von Nukleinsäuren in einer Probe, umfassend:
1. Amplifizierung von KIF11-Nukleinsäuren, die in der Probe umfasst sind, unter Verwendung eines ersten und eines zweiten KIF11-spezifischen Primerpaares in einer KIF11-Amplifikationsreaktion,
a. wobei jeder der Bestandteile des ersten KIF11-spezifischen Primerpaares - unabhängig voneinander - komplementär zu einer KIF11-Region ist, die in einem Exon, Intron oder der nicht-kodierenden Sequenz des KIF11-Gens lokalisiert ist;
b. wobei die Amplifikationsreaktion, die das erste KIF11-spezifische Primerpaar verwendet, in Anwesenheit einer ersten KIF11-nachweisbaren Sonde durchgeführt wird;
c. wobei jeder der Bestandteile des zweiten KIF11-spezifischen Primerpaares - unabhängig voneinander - komplementär zu einer KIF11-Region ist, die in einem Exon, Intron oder der nicht-kodierenden Sequenz des KIF11-Gens lokalisiert ist;
d. wobei die Amplifikationsreaktion, die das zweite KIF11-spezifische Primerpaar verwendet, in Anwesenheit einer zweiten KIF11-nachweisbaren Sonde durchgeführt wird;
2. Bestimmung der Schwellenwerte in jeder der Nukleinsäure-Amplifikationsreaktionen durch
i. Messen zu mehreren unterschiedlichen Zeitpunkten während der Amplifikationsreaktion mindestens eines Signals, dessen Intensität im Verhältnis zu der Anzahl einer in der Reaktion amplifizierten Nukleinsäuresequenz steht; und
ii. Bestimmung der Zykluszahl, die mit dem Merkmal des Derivates assoziiert ist, welche den Schwellenwert darstellt;
3. Vergleich der Schwellenwerte (Schwellenzykluszahlen) der ersten und zweiten KIF11-Amplifikationsreaktionen;
4. wobei die Differenz zwischen den Schwellenzykluszahlen (ΔCq) der ersten und der zweiten KIF11-Amplifikationsreaktionen ein Maß für die Integrität der genomischen DNA ist.

13. Verfahren nach Anspruch 12, wobei die durch die Amplifikationsreaktion des ersten und zweiten KIF11-spezifischen Primerpaares gebildeten Amplicons ausreichend weit lokalisiert sind, um nicht zu interferieren.

14. Verfahren nach Anspruch 12 oder 13, wobei die Amplicons mindestens 600 Basenpaare (bp), wie etwa mindestens 700 bp, 800 bp, 900 bp oder sogar mehr, wie etwa mindestens 1 Kilobasenpaare sind.

15. Verfahren zur gDNA-Fragmentierungsbestimmung, umfassend
i. Erzeugung 3er (erster, zweiter und dritter) KIF11-Amplicons von feststellbarer Länge durch erste, zweite und dritte Amplifikationsreaktionen, bevorzugt durch PCR;
ii. Bestimmung der Cq-Werte der ersten, zweiten und dritten Amplifikationsreaktionen;
iii. Vergleich der Cq der ersten, zweiten und dritten Amplifikationsreaktionen;
wobei die Differenz der Cq-Werte der ersten, zweiten und dritten Amplifikationsreaktionen eine Indikation der gDNA-Fragmentierung ist; oder
i. Erzeugung eines ersten KIF11-Amplicons ("kurz") durch eine erste Amplifikationsreaktion;
ii. Erzeugung eines zweiten KIF11-Amplicons ("lang") durch eine zweite Amplifikationsreaktion;
iii. Bestimmung der Cq-Werte der ersten und der zweiten Amplifikationsreaktion;
iv. Bestimmung des ΔCq-Wertes der ersten und der zweiten Amplifikationsreaktion;
wobei die ΔCq eine Indikation der gDNA-Fragmentierung ist.

16. Verfahren zur Qualitätskontrolle des Prozessierungs-, Isolierungs-und Amplifikationsprozesses, umfassend:
i. Probenprozessierung;
ii. Nukleinsäureisolierung;
iii. Erzeugung 3er KIF11-Amplicons von feststellbarer Länge durch Amplifikationsreaktionen;
iv. Bestimmung der Cq-Werte von jedem der KIF11-Amplicons;
wobei die Cq-Werte der KIF11-Amplicons ein Maß für die Qualitätskontrolle der Prozessierung, Isolierung und Amplifikation sind.

17. Verfahren nach einem der Ansprüche 1-9, umfassend mindestens einen Primer von den Ziel-spezifischen Primerpaaren, ausgewählt aus den folgenden:
(1) ein EGFR Primerpaar, wobei der
EGFR Forward Primer ausgewählt ist aus SEQ ID NO:s 1-34, und
EGFR Reverse Primer ausgewählt ist aus SEQ ID NO:s 44-48;
bevorzugt wird das EGFR-Amplicon durch eins der SEQ ID NO:s 53-57 detektiert;
(2) ein BRAF Primerpaar, wobei der
BRAF Forward Primer ausgewählt ist aus SEQ ID NO:s 35 und 36, und
BRAF Reverse Primer ist SEQ ID NO:s 49;
bevorzugt wird das BRAF-Amplicon durch SEQ ID NO:s 58 detektiert;
(3) ein KRAS Primerpaar, wobei der
KRAS Forward Primer ausgewählt ist aus SEQ ID NO:s 37-39 und 67-68, und
KRAS Reverse Primer ist SEQ ID NO:s 50;
bevorzugt wird das KRAS-Amplicon durch SEQ ID NO:s 59 detektiert;
(4) ein HER2 Primerpaar, wobei der
HER2 Forward Primer ausgewählt ist aus SEQ ID NO:s 40-42, und
HER2 Reverse Primer ist SEQ ID NO:s 51;
bevorzugt wird das HER2-Amplicon durch SEQ ID NO:s 60 detektiert.

## Revendications

1. - Procédé de quantification du nombre d'acides nucléiques cibles dans un échantillon par rapport aux acides nucléiques de KIF11 dans ledit échantillon, comprenant :
1. amplifier les acides nucléiques de KIF11 compris dans l'échantillon à l'aide d'une paire d'amorces spécifiques de KIF11 dans une réaction d'amplification de KIF11,
i. dans lequel chacun des membres de la paire d'amorces spécifiques de KIF11 est - indépendamment l'un de l'autre - complémentaire d'une région de KIF11 située dans un exon, un intron ou la séquence non codante du gène KIF11 ;
ii. dans lequel la réaction d'amplification de KIF11 est effectuée en présence d'une sonde détectable de KIF11 ;
2. détecter un signal produit par la sonde détectable de KIF11 dans la réaction d'amplification de KIF11 ;
3. quantifier le signal de 2. ;
4. amplifier les acides nucléiques cibles de l'échantillon à l'aide d'une paire d'amorces spécifiques de la cible dans une réaction d'amplification de la cible ;
dans lequel la réaction d'amplification de la cible est effectuée en présence d'une sonde détectable de la cible ; et
5. détecter un signal produit par la sonde détectable de la cible dans la réaction d'amplification de la cible ;
6. quantifier le signal de 5. ;
7. normaliser le signal quantifié de 6. au signal quantifié de 3., quantifiant ainsi le nombre d'acides nucléiques cibles dans un échantillon par rapport aux acides nucléiques de KIF11 dans l'échantillon.

2. - Procédé selon la revendication 1, dans lequel ladite région de KIF11 est située dans l'un quelconque des exons 6, 8, 18, 21, de l'intron 6, de la limite exon 21 - intron 21 ou d'une région non codante du gène KIF11.

3. - Procédé selon l'une des revendications 1 ou 2, dans lequel la paire d'amorces spécifiques de KIF est une paire d'amorces choisie parmi les suivantes :
i. SEQ ID NO: 69 et 70 ;
ii. SEQ ID NO: 71 et 72 ;
iii. SEQ ID NO: 73 et 74 ;
iv. SEQ ID NO: 75 et 76 ;
v. SEQ ID NO: 77 et 78 ; et
vi. SEQ ID NO: 79 et 80.

4. - Procédé selon l'une quelconque des revendications 1 à 3, dans lequel la sonde détectable de KIF11 est choisie parmi SEQ ID NO: 81 à 88.

5. - Procédé selon l'une quelconque des revendications 1 à 4, dans lequel les sondes sont marquées par fluorescence.

6. - Procédé selon l'une quelconque des revendications 1 à 5, dans lequel l'acide nucléique cible et le gène KIF11 sont amplifiés dans la même réaction d'amplification et/ou obtenus à partir du même échantillon.

7. - Procédé selon l'une quelconque des revendications 1 à 6, dans lequel la réaction d'amplification est une réaction en chaîne par polymérase (PCR), de préférence une PCR quantitative (qPCR).

8. - Procédé selon la revendication 7, dans lequel le résultat de la qPCR est rapporté en tant que valeur ΔCq ou valeur ΔCq double.

9. - Procédé selon l'une quelconque des revendications 1 à 8, dans lequel l'échantillon est un échantillon de tissu frais ou un échantillon congelé ou FFPE.

10. - Utilisation pour amplifier une région du gène de référence génomique KIF11 d'un kit comprenant des amorces et des instructions comprenant un protocole d'amplification et d'analyse des résultats, dans laquelle les amorces sont une paire d'amorces choisies parmi les suivantes :
i. SEQ ID NO: 69 et 70 ;
ii. SEQ ID NO: 71 et 72 ;
iii. SEQ ID NO: 73 et 74 ;
iv. SEQ ID NO: 75 et 76 ;
v. SEQ ID NO: 77 et 78 ; et
vi. SEQ ID NO: 79 et 80,
de préférence la région amplifiée est détectée par des sondes choisies parmi SEQ ID NO: 81 à 86, de préférence par l'intermédiaire d'un rapporteur générique choisi parmi SEQ ID NO: 87 et 88.

11. - Procédé de détermination de contamination de l'ADNg dans un échantillon, comprenant :
1. amplifier l'acide nucléique de KIF11 compris dans l'échantillon à l'aide d'une première et d'une deuxième paires d'amorces spécifiques de KIF11 dans une réaction d'amplification de KIF11,
a. dans lequel au moins un membre de la première paire d'amorces spécifiques de KIF11 est complémentaire d'un intron de KIF11 ou d'une séquence non codante du gène KIF11 ;
b. dans lequel la réaction d'amplification à l'aide de la première paire d'amorces spécifiques de KIF11 est effectuée en présence d'une première sonde détectable de KIF11 ;
c. dans lequel chacun des membres de la deuxième paire d'amorces spécifiques de KIF11 est situé dans un exon de KIF11 ;
d. dans lequel la réaction d'amplification à l'aide de la deuxième paire d'amorces spécifiques de KIF11 est effectuée en présence d'une deuxième sonde détectable de KIF11 ;
2. détecter des signaux produits par la première et la deuxième sondes détectables de KIF11 dans les première et deuxième réactions d'amplification de KIF11 ;
3. quantifier les signaux des première et deuxième réactions d'amplification de KIF11 ;
4. normaliser le signal quantifié de la première réaction d'amplification au signal quantifié du deuxième signal d'amplification, déterminant ainsi une contamination de l'ADNg dans l'échantillon, de préférence l'échantillon est un échantillon d'ADN mitochondrial, d'ADNc, d'ARNm, d'ARNr, d'ARNt, d'ARNnh, de microARN, d'ARNlnc, d'ADNcf, d'ADN tumoral acellulaire ou d'ARNsi.

12. - Procédé de détermination de l'intégrité des acides nucléiques dans un échantillon, comprenant :
1. amplifier l'acide nucléique de KIF11 compris dans l'échantillon à l'aide d'une première et d'une deuxième paires d'amorces spécifiques de KIF11 dans une réaction d'amplification de KIF11,
a. dans lequel chacun des membres de la première paire d'amorces spécifiques de KIF11 est - indépendamment l'un de l'autre - complémentaire d'une région de KIF11 située dans un exon, un intron ou la séquence non codante du gène KIF11 ;
b. dans lequel la réaction d'amplification à l'aide de la première paire d'amorces spécifiques de KIF11 est effectuée en présence d'une première sonde détectable de KIF11 ;
c. dans lequel chacun des membres de la deuxième paire d'amorces spécifiques de KIF11 est - indépendamment l'un de l'autre - complémentaire d'une région de KIF11 située dans un exon, un intron ou la séquence non codante du gène KIF11 ;
d. dans lequel la réaction d'amplification à l'aide de la deuxième paire d'amorces spécifiques à KIF11 est effectuée en présence d'une deuxième sonde détectable de KIF11 ;
2. déterminer les valeurs seuil dans chacune desdites réactions d'amplification d'acide nucléique par
i. mesure, à une pluralité de moments différents pendant la réaction d'amplification, d'au moins un signal dont l'intensité est liée à la quantité d'une séquence d'acide nucléique en cours d'amplification dans la réaction ; et
ii. détermination du nombre de cycles associé à la caractéristique du dérivé, qui représente la valeur seuil ;
3. comparer les valeurs seuil (nombres de cycles seuil) des première et deuxième réactions d'amplification de KIF11 ;
4. dans lequel la différence entre les nombres de cycles seuil (ΔCq) des première et deuxième réactions d'amplification de KIF11 est une mesure pour l'intégrité de l'ADN génomique.

13. - Procédé selon la revendication 12, dans lequel les amplicons générés par la réaction d'amplification des première et deuxième paires d'amorces spécifiques de KIF11 sont suffisamment éloignées pour ne pas interférer.

14. - Procédé selon l'une des revendications 12 ou 13, dans lequel les amplicons sont d'au moins 600 paires de bases (pb), tel qu'au moins 700 pb, 800 pb, 900 pb ou même plus, tel qu'au moins 1 kilo paires de bases.

15. - Procédé de détermination de la fragmentation de l'ADNg, comprenant
i. générer 3 (premier, deuxième et troisième) amplicons de KIF11 de longueurs discernables par des première, deuxième et troisième réactions d'amplification, de préférence par PCR ;
ii. déterminer les valeurs Cq desdites première, deuxième et troisième réactions d'amplification ;
iii. comparer les Cq desdites première, deuxième et troisième réactions d'amplification ;
dans lequel les différences dans les valeurs Cq entre lesdites première, deuxième et troisième réactions d'amplification sont une indication de la fragmentation de l'ADNg ; ou
i. générer un premier amplicon de KIF11 (« court ») par une première réaction d'amplification ; et
ii. générer un deuxième amplicon de KIF11 (« long ») par une deuxième réaction d'amplification ;
iii. déterminer les valeurs Cq desdites première et deuxième réactions d'amplification ;
iv. déterminer la valeur ΔCq de ladite première et de ladite deuxième réaction d'amplification ;
dans lequel ΔCq est une indication de la fragmentation de l'ADNg.

16. - Procédé de contrôle qualité pour le processus de traitement, d'isolement et d'amplification comprenant :
i. le traitement d'un échantillon ;
ii. l'isolement d'un acide nucléique ;
iii. la génération de 3 amplicons de KIF11 de tailles différentes de manière perceptible par des réactions d'amplification ;
iv. la détermination des valeurs Cq de chacun desdits amplicons de KIF11 ;
dans lequel les valeurs Cq des amplicons de KIF11 sont une mesure du contrôle qualité du traitement, de l'isolement et de l'amplification.

17. - Procédé selon l'une quelconque des revendications 1 à 9, comprenant au moins une amorce parmi les paires d'amorces spécifiques de la cible choisie parmi les suivantes :
(1) une paire d'amorces d'EGFR, où
l'amorce sens d'EGFR est choisie parmi SEQ ID NO: 1 à 34, et
l'amorce antisens d'EGFR est choisie parmi SEQ ID NO: 44 à 48 ;
de préférence l'amplicon d'EGFR est détecté par l'une quelconque de SEQ ID NO: 53 à 57 ;
(2) une paire d'amorces de BRAF, où
l'amorce sens de BRAF est choisie parmi SEQ ID NO: 35 et 36, et
l'amorce antisens de BRAF est SEQ ID NO: 49 ;
de préférence l'amplicon de BRAF est détecté par SEQ ID NO: 58 ;
(3) une paire d'amorces de KRAS, où l'amorce sens de KRAS est choisie parmi SEQ ID NO:
37 à 39 et 67 à 68, et
l'amorce antisens de KRAS est SEQ ID NO: 50 ;
de préférence l'amplicon de KRAS est détecté par SEQ ID NO: 59 ;
(4) une paire d'amorces de HER2, où
l'amorce sens de HER2 est choisie parmi SEQ ID NO: 40 à 42, et
l'amorce antisens de HER2 est SEQ ID NO: 51 ;
de préférence l'amplicon de HER2 est détecté par SEQ ID NO: 60.
